(19) Europäisches Patentamt
European Patent Office
Office européen des brevets

(11) Publication number : 0 500 210 A2

# (12) EUROPEAN PATENT APPLICATION

(21) Application number : 92300470.9

(22) Date of filing : 20.01.92

(51) Int. Cl.[5] : **C09K 19/34,** C09K 19/18, C09K 19/14, C09K 19/42, C07D 213/24, C07D 213/63, C07C 43/225, C07D 239/26, C07D 239/34, C07D 237/08, C07D 237/14

(30) Priority : 19.01.91 JP 19425/91
03.06.91 JP 159789/91
09.10.91 JP 290859/91

(43) Date of publication of application :
26.08.92 Bulletin 92/35

(84) Designated Contracting States :
DE FR GB IT

(71) Applicant : SANYO CHEMICAL INDUSTRIES, LTD.
11-1, Ichinohashinomoto-cho, Higashiyama-ku
Kyoto-shi 605 (JP)

(72) Inventor : Sugita, Naoko
12-44 Higashinominamiinoue-Cho
Yamashina-ku, Kyoto (JP)
Inventor : Satoh, Masahiro
Corporouse Fushimi 112,32
Fukakusa-Ikenouchi-Cho
Fushimi-ku, Kyoto (JP)
Inventor : Watanabe, Tetsuya
71-38 Muranohigashi-machi
Hirakata-shi, Osaka-fu (JP)
Inventor : Yoshio, Kunikiyo
1922-361, Noji-cho
Kasatsu, Shiga-ken (JP)
Inventor : Yanagi, Tatsuroh
10-1 Imakumano-minamihiyoshi-cho
Higashiyama-ku, Kyoto (JP)

(74) Representative : Marlow, Nicholas Simon
Reddie & Grose 16, Theobalds Road
London WC1X 8PL (GB)

## (54) Liquid crystal compounds and compositions.

(57) Smectic C liquid crystal compounds, represented by the following formula (1) or (2) are disclosed.

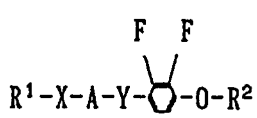

(1)

$R^1$-O-$A^1$-C≡C-$A^2$-CO-$(O)_p$-$R^2$ (2)

wherein $R^1$ and $R^2$ are C1-18 alkyl groups ; A is a cyclic group, such as substituted or unsubstituted 1,4-phenylene group,

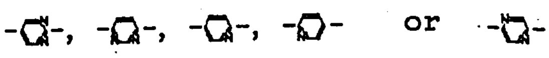

X is direct bond, O or C≡C ; Z is -C≡C- or $-CH_2CH_2-$ ; one of $A^1$ and $A^2$ is 1,4-phenylene group and the other is 2,3-difluoro-1,4-phenylene group ; and p is 0 or 1.

These compounds can provide ferroelectric liquid crystal composition of low viscosity, and are useful as a component giving negative dielectric anisotropy and enlarging a temperature range showing smectic C phase.

EP 0 500 210 A2

## BACKGROUND OF THE INVENTION

### 1. Field of the Invention

This invention relates to liquid crystal compounds and liquid crystal compositions, useful as ferroelectric liquid crystal materials.

### 2. Description of the Prior Art

As liquid crystal compounds showing smectic C phase or ciral smectic C phase, for used in ferroelectric liquid crystal materials, there have been known liquid crystal phenyl pyrimidine compounds, such as 5-alkyl-2-(4′-alkoxyphenyl)pyrimidines.

However, these liquid crystal compounds are of positive dielectric anisotropy, and therefore improvement of memory or storage capacity is not attained even when is used a driving method of liquid crystal display elements called AC stabilize method.

## SUMMARY OF THE INVENTION

It has been desired to provide a liquid crystal compound showing smectic phase suitable for such as driving method.

It has also been desired to provide a liquid crystal compound capable of providing ferroelectric liquid crystal composition of low viscosity.

It has also been desired to provide a liquid crystal compound, useful as a component giving negative dielectric anisotropy to liquid crystal composition.

It has also been desired to provide a liquid crystal compound, showing smectic C phase over a wide temperature range.

The present invention provides liquid crystal compounds represented by the following general formula (1) or (2):

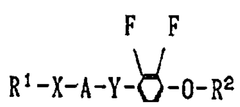

(1)

$$R^1\text{-O-}A^1\text{-C}\equiv\text{C-}A^2\text{-CO-(O)}_p\text{-}R^2 \qquad (2)$$

wherein $R^1$ and $R^2$ are the same or different alkyl groups containing 1 to 18 carbon atoms, which are each unsubstituted or are each substituted with at least one subsituent selected from the group consisting of F, Cl, alkoxy, trifluoromethyl and cyano groups;

A is a cyclic group selected from the group consisting of Ph, $X^1$, $X^2$, $X^3$, $X^4$, $X^5$, Ph-$X^4$ and $X^1$-Ph, wherein Ph represents 1,4-phenylene group, which is unsubstituted or substituted with at least one subsituent selected from the group consisting of F, Cl, CN and $NO_2$; $X^1$, $X^2$, $X^3$, $X^4$ and $X^5$ represent

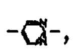, 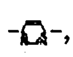, 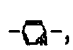, 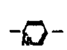

and

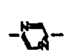 ,

respectively;

X is direct bond, O or -C≡C-, X being O when A is Ph;

Z is -C≡C- or $-CH_2CH_2-$;

one of $A^1$ and $A^2$ is 1,4-phenylene group and the other is 2,3-difluoro-1,4-phenylene group; and p is 0 or 1.

## BRIEF DESCRIPTION OF THE DRAWING

Fig.1(a) to Fig.4(a) are IR spectra of compounds of Examples 1 to 4, respectively. Fig.1(b) to Fig.4(b) are H-NMR spectra of compounds of Examples 1 to 4, respectively. Fig.1(c) to Fig.4(c) are F-NMR spectra of compounds of Examples 1 to 4, respectively.

DETAILED DESCRIPTION OF THE PREFERRED EMBODIMENTS

In the general formulae (1) and (2), $R^1$ and $R^2$ are the same or different alkyl groups containing usually 1 - 18 carbon atoms, which may be substituted with at least one substituent selected from the group consisting of fluorine, chlorine, alkoxy groups containing 1 to 10 or more carbon atoms, trifluoromethyl group and cyano group.

Illustrative of suitable alkyl groups are straightchain alkyl groups, branched alkyl groups, cyclic alkyl groups, F-substituted alkyl groups, Cl-substituted alkyl groups and alkoxy-substituted alkyl groups, as written in EP 0 385 692 as examples of R and R′; trifluoromethyl-substituted alkyl groups, such as 1-trifluoromethylpropyl, 1-trifluoromethylbutyl, 1-trifluoromethylpentyl, 1-trifluoromethylhexyl, 1-trifluoromethylheptyl, 1-trifluoromethyloctyl, 1-trifluoromethylnonyl, 1-trifluoromethyldecyl, 1-trifluoromethylundecyl, 1-trifluoromethyldodecyl, 1-trifluoromethyl-2-methoxyethyl, 1-trifluoromethyl-2-ethoxyethyl, 1-trifluoromethyl-2-propoxyethyl, 1-trifluoromethyl-2-butoxyethyl, 1-trifluoromethyl-2-pentyloxyethyl, 1-trifluoromethyl-2-hexyloxyethyl, 1-trifluoromethyl-2-isopropyloxyethyl, 1-trifluoromethyl-2-(1′-methylpropyloxy)ethyl, 1-trifluoromethyl-2-(1′-methylbutyloxy)ethyl, 1-trifluoromethyl-2-(1′-methylpentyloxy)ethyl, 1-trifluoromethyl-2-(1′-methylhexyloxy)ethyl, 1-trifluoromethyl-2-(1′-methylheptyloxy)ethyl, 1-trifluoromethyl-2-(2′-methylbutyloxy)ethyl, 1-trifluoromethyl-3-methoxypropyl, 1-trifluoromethyl-3-ethoxypropyl, 1-trifluoromethyl-3-propoxypropyl, 1-trifluoromethyl-3-butyloxypropyl, 1-trifluoromethyl-3-pentyloxypropyl, 1-trifluoromethyl-3-hexyloxypropyl, 1-trifluoromethyl-3-iso-propyloxypropyl, 1-trifluoromethyl-3-(1′-methylpropyloxy)propyl, 1-trifluoromethyl-3-(1′-methylbutyloxy)propyl, 1-trifluoromethyl-3-(1′-methylpentyloxy)propyl, 1-trifluoromethyl-3-(1′-methylhexyloxy)propyl, 1-trifluoromethyl-3-(1′-methylheptyloxy)propyl, 1-trifluoromethyl-3-(2′-methylbutyloxy)propyl, 1-trifluoromethyl-4-methoxybutyl, 1-trifluoromethyl-4-ethoxybutyl, 1-trifluoromethyl-4-propyloxybutyl, 1-trifluoromethyl-4-butyloxybutyl, 1-trifluoromethyl-4-pentyloxybutyl, 1-trifluoromethyl-4-hexyloxybutyl, 1-trifluoromethyl-4-iso-propyloxybutyl, 1-trifluoromethyl-4-(1′methylpropyloxy)butyl, 1-trifluoromethyl-4-(1′-methylbutyloxy)butyl, 1-trifluoromethyl-4-(1′-methylpentyloxy) butyl, 1-trifluoromethyl-4-(1′-methylhexyloxy)butyl, 1-trifluoromethyl-4-(1′-methylheptyloxy)butyl, and 1-trifluoromethyl-4-(2′-methylbutyloxy)butyl; and cyano-substituted alkyl groups, such as 1-cyanopropyl, 1-cyanobutyl, 1-cyanopentyl, 1-cyanohexyl, 1-cyanoheptyl, 1-cyanooctyl, 1-cyanononyl, 1-cyanodecyl, 1-cyanoundecyl and 1-cyanododecyl.

Alkyl groups R and R′ may contain one or more asymmetric carbon atoms, and may be optically active (hereinafter referred to as oa). Illustrative of suitable oa alkyl groups are oa alkyl groups, F-substituted oa alkyl groups, Cl-substituted oa alkyl groups and alkoxy-substituted oa alkyl groups, as written in EP 0 385 692 as examples of R and R′; as well as oa ones among trifluoromethyl-substituted alkyl groups and cyano-substituted as mentioned hereinabove.

Among these, preferred are alkyl groups containing 1 to 16, particularly 1 to 14 carbon atoms.

In the formula (2), among alkyl groups of $R^2$, preferred are asymmetric carbon atom-containing alkyl groups of the formula: $-(CH_2)_nC^*H(CH_3)R'$, wherein R′ is an alkyl group containing 2-10 (particularly 2-6) carbon atoms and n is an integer of 0-9 (particularly 0-5).

In the general formula (1), A is a cyclic group selected from the group consisting of Ph, $X^1$, $X^2$, $X^3$, $X^4$, $X^5$, Ph-$X^4$ and $X^1$-Ph. Suitable examples of Ph include 1,4-phenylene group, and substituted 1,4-phenylene groups, such as ones substituted with 1-4 fluorine atoms, ones substituted with 1 or 2 substituents selected from Cl, Br, CN and $NO_2$. Among A′s, preferred are 1,4-phenylene group, F-substituted 1,4-phenylene groups, $X^1$, $X^2$, $X^3$, $X^4$, $X^5$, Ph-$X^4$ and $X^1$-Ph.

In the general formula (1), X is - (direct bond), O (ether oxygen linkage) or -C≡C-, X being O when A is Ph; and Z is -C≡C- or $-CH_2CH_2-$.

Illustrative examples of the compounds represented by the formula (1) or (2) include those shown in Table 1 to Table 11. In these Tables and also hereinafter, are used the following abbreviated words:

MET: $CH_3-$;

ETY: $C_2H_5-$;

PRO: $n-C_3H_7-$;

BUT: $n-C_4H_9-$;

PEN: $n-C_5H_{11}-$;

HEX: $n-C_6H_{13}-$;

HEP: $n-C_7H_{15}-$;

OCT: $n-C_8H_{17}-$;

NON: $n-C_9H_{19}-$;

DEC: $n-C_{10}H_{21}-$;

2MB: $C_2H_5C^*HCH_2-$;
$\quad\quad /$
$\quad CH_3$

4MH: $C_2H_5C^*HCH_2CH_2CH_2-$;
$\quad\quad /$
$\quad CH_3$

2EOPr: $C_2H_5OC^*HCH_2-$;
$\quad\quad\quad /$
$\quad\quad CH_3$

$CF_3$HEX: $n-C_6H_{13}C^*H-$;
$\quad\quad\quad /$
$\quad\quad CF_3$

$F_2$Ph: 2,3-difluoro-1,4-phenylene

Table 1. Formula (1) wherein A is Ph and Y is -C≡C-

| No. | $R^1$ | X | $R^2$ | No. | $R^1$ | X | $R^2$ | No. | $R^1$ | X | $R^2$ |
|---|---|---|---|---|---|---|---|---|---|---|---|
| 1 | PEN | O | ETY | 10 | OCT | O | OCT | 19 | DEC | O | HEP |
| 2 | HEX | O | HEP | 11 | OCT | O | NON | 20 | DEC | O | OCT |
| 3 | HEP | O | HEX | 12 | OCT | O | DEC | 21 | DEC | O | NON |
| 4 | HEP | O | HEP | 13 | NON | O | HEX | 22 | DEC | O | DEC |
| 5 | HEP | O | OCT | 14 | NON | O | HEP | 23 | DEC | O | 2MB |
| 6 | HEP | O | NON | 15 | NON | O | OCT | 24 | DEC | O | 4MH |
| 7 | HEP | O | DEC | 16 | NON | O | NON | 25 | DEC | O | 2EPOr |
| 8 | OCT | O | HEX | 17 | NON | O | DEC | 26 | DEC | O | $CF_3$HEX |
| 9 | OCT | O | HEP | 18 | DEC | O | HEX | | | | |

Table 2. Formula (1) wherein A is $X^4$ and Y is $-C\equiv C-$

| No. | $R^1$ | X | $R^2$ | No. | $R^1$ | X | $R^2$ | No. | $R^1$ | X | $R^2$ |
|---|---|---|---|---|---|---|---|---|---|---|---|
| 27 | PEN | O | MET | 37 | HEP | O | DEC | 47 | NON | O | DEC |
| 28 | PEN | O | ETY | 38 | OCT | O | HEX | 48 | DEC | O | HEX |
| 29 | PEN | O | PRO | 39 | OCT | O | HEP | 49 | DEC | O | HEP |
| 30 | HEX | O | BUT | 40 | OCT | O | OCT | 50 | DEC | O | OCT |
| 31 | HEX | O | HEX | 41 | OCT | O | NON | 51 | DEC | O | NON |
| 32 | HEX | O | HEP | 42 | OCT | O | DEC | 52 | DEC | O | DEC |
| 33 | HEX | O | OCT | 43 | NON | O | HEX | 53 | DEC | O | 2MB |
| 34 | HEP | O | HEP | 44 | NON | O | HEP | 54 | DEC | O | 4MH |
| 35 | HEP | O | OCT | 45 | NON | O | OCT | 55 | DEC | O | 2EPOr |
| 36 | HEP | O | NON | 46 | NON | O | NON | 56 | DEC | O | $CF_3$HEX |

Table 2. (continued)

| No. | $R^1$ | X | $R^2$ | No. | $R^1$ | X | $R^2$ | No. | $R^1$ | X | $R^2$ |
|---|---|---|---|---|---|---|---|---|---|---|---|
| 57 | PEN | – | MET | 77 | NON | – | DEC | 97 | HEP | C≡C | DEC |
| 58 | PEN | – | ETY | 78 | DEC | – | HEX | 98 | OCT | C≡C | HEX |
| 59 | PEN | – | PRO | 79 | DEC | – | HEP | 99 | OCT | C≡C | HEP |
| 60 | HEX | – | BUT | 80 | DEC | – | OCT | 100 | OCT | C≡C | OCT |
| 61 | HEX | – | HEX | 81 | DEC | – | NON | 101 | OCT | C≡C | NON |
| 62 | HEX | – | HEP | 82 | DEC | – | DEC | 102 | OCT | C≡C | DEC |
| 63 | HEX | – | OCT | 83 | DEC | – | 2MB | 103 | NON | C≡C | HEX |
| 64 | HEP | – | HEP | 84 | DEC | – | 4MH | 104 | NON | C≡C | HEP |
| 65 | HEP | – | OCT | 85 | DEC | – | 2EOPr | 105 | NON | C≡C | OCT |
| 66 | HEP | – | NON | 86 | DEC | – | $CF_3$HEX | 106 | NON | C≡C | NON |
| 67 | HEP | – | HEX | 87 | PEN | C≡C | MET | 107 | NON | C≡C | DEC |
| 68 | OCT | – | HEX | 88 | PEN | C≡C | ETY | 108 | DEC | C≡C | HEX |
| 69 | OCT | – | HEP | 89 | PEN | C≡C | PRO | 109 | DEC | C≡C | HEP |
| 70 | OCT | – | OCT | 90 | HEX | C≡C | BUT | 110 | DEC | C≡C | OCT |
| 71 | OCT | – | NON | 91 | HEX | C≡C | HEX | 111 | DEC | C≡C | NON |
| 72 | OCT | – | DEC | 92 | HEX | C≡C | HEP | 112 | DEC | C≡C | DEC |
| 73 | NON | – | HEX | 93 | HEX | C≡C | OCT | 113 | DEC | C≡C | 2MB |
| 74 | NON | – | HEP | 94 | HEP | C≡C | HEP | 114 | DEC | C≡C | 4MH |
| 75 | NON | – | OCT | 95 | HEP | C≡C | OCT | 115 | DEC | C≡C | 2EPOr |
| 76 | NON | – | NON | 96 | HEP | C≡C | NON | 116 | DEC | C≡C | $CF_3$HEX |

Table 3. Formula (1) wherein A is $X^3$ and Y is -C≡C-

| No. | $R^1$ | X | $R^2$ | No. | $R^1$ | X | $R^2$ | No. | $R^1$ | X | $R^2$ |
|---|---|---|---|---|---|---|---|---|---|---|---|
| 117 | PEN | O | MET | 138 | NON | O | DEC | 159 | HEP | – | DEC |
| 118 | PEN | O | ETY | 139 | DEC | O | HEX | 160 | OCT | – | HEX |
| 119 | PEN | O | PRO | 140 | DEC | O | HEP | 161 | OCT | – | HEP |
| 120 | HEX | O | BUT | 141 | DEC | O | OCT | 162 | OCT | – | OCT |
| 121 | HEX | O | HEX | 142 | DEC | O | NON | 163 | OCT | – | NON |
| 122 | HEX | O | HEP | 143 | DEC | O | DEC | 164 | OCT | – | DEC |
| 123 | HEX | O | OCT | 144 | DEC | O | 2MB | 165 | NON | – | HEX |
| 124 | HEP | O | HEX | 145 | DEC | O | 4MH | 166 | NON | – | HEP |
| 125 | HEP | O | HEP | 146 | DEC | O | 2EPOr | 167 | NON | – | OCT |
| 126 | HEP | O | OCT | 147 | DEC | O | $CF_3$HEX | 168 | NON | – | NON |
| 127 | HEP | O | NON | 148 | PEN | – | MET | 169 | NON | – | DEC |
| 128 | HEP | O | DEC | 149 | PEN | – | ETY | 170 | DEC | – | HEX |
| 129 | OCT | O | HEX | 150 | PEN | – | PRO | 171 | DEC | – | HEP |
| 130 | OCT | O | HEP | 151 | HEX | – | BUT | 172 | DEC | – | OCT |
| 131 | OCT | O | OCT | 152 | HEX | – | HEX | 173 | DEC | – | NON |
| 132 | OCT | O | NON | 153 | HEX | – | HEP | 174 | DEC | – | DEC |
| 133 | OCT | O | DEC | 154 | HEX | – | OCT | 175 | DEC | – | 2MB |
| 134 | NON | O | HEX | 155 | HEP | – | HEX | 176 | DEC | – | 4MH |
| 135 | NON | O | HEP | 156 | HEP | – | HEP | 177 | DEC | – | 2EOPr |
| 136 | NON | O | OCT | 157 | HEP | – | OCT | 178 | DEC | – | $CF_3$HEX |
| 137 | NON | O | NON | 158 | HEP | – | NON | | | | |

Table 4. Formula (1) wherein A is $X^1$ and Y is -C≡C-

| No. | $R^1$ | X | $R^2$ | No. | $R^1$ | X | $R^2$ | No. | $R^1$ | X | $R^2$ |
|---|---|---|---|---|---|---|---|---|---|---|---|
| 179 | PEN | O | MET | 200 | NON | O | DEC | 221 | HEP | – | DEC |
| 180 | PEN | O | ETY | 201 | DEC | O | HEX | 222 | OCT | – | HEX |
| 181 | PEN | O | PRO | 202 | DEC | O | HEP | 223 | OCT | – | HEP |
| 182 | HEX | O | BUT | 203 | DEC | O | OCT | 224 | OCT | – | OCT |
| 183 | HEX | O | HEX | 204 | DEC | O | NON | 225 | OCT | – | NON |
| 184 | HEX | O | HEP | 205 | DEC | O | DEC | 226 | OCT | – | DEC |
| 185 | HEX | O | OCT | 206 | DEC | O | 2MB | 227 | NON | – | HEX |
| 186 | HEP | O | HEX | 207 | DEC | O | 4MH | 228 | NON | – | HEP |
| 187 | HEP | O | HEP | 208 | DEC | O | 2EPOr | 229 | NON | – | OCT |
| 188 | HEP | O | OCT | 209 | DEC | O | $CF_3$HEX | 230 | NON | – | NON |
| 189 | HEP | O | NON | 210 | PEN | – | MET | 231 | NON | – | DEC |
| 190 | HEP | O | DEC | 211 | PEN | – | ETY | 232 | DEC | – | HEX |
| 191 | OCT | O | HEX | 212 | PEN | – | PRO | 233 | DEC | – | HEP |
| 192 | OCT | O | HEP | 213 | HEX | – | BUT | 234 | DEC | – | OCT |
| 193 | OCT | O | OCT | 214 | HEX | – | HEX | 235 | DEC | – | NON |
| 194 | OCT | O | NON | 215 | HEX | – | HEP | 236 | DEC | – | DEC |
| 195 | OCT | O | DEC | 216 | HEX | – | OCT | 237 | DEC | – | 2MB |
| 196 | NON | O | HEX | 217 | HEP | – | HEX | 238 | DEC | – | 4MH |
| 197 | NON | O | HEP | 218 | HEP | – | HEP | 239 | DEC | – | 2EOPr |
| 198 | NON | O | OCT | 219 | HEP | – | OCT | 240 | DEC | – | $CF_3$HEX |
| 199 | NON | O | NON | 220 | HEP | – | NON | | | | |

Table 5. Formula (1) wherein A is $X^2$ and Y is $-C \equiv C-$

| No. | $R^1$ | X | $R^2$ | No. | $R^1$ | X | $R^2$ | No. | $R^1$ | X | $R^2$ |
|---|---|---|---|---|---|---|---|---|---|---|---|
| 241 | PEN | O | MET | 262 | NON | O | DEC | 283 | HEP | – | DEC |
| 242 | PEN | O | ETY | 263 | DEC | O | HEX | 284 | OCT | – | HEX |
| 243 | PEN | O | PRO | 264 | DEC | O | HEP | 285 | OCT | – | HEP |
| 244 | HEX | O | BUT | 265 | DEC | O | OCT | 286 | OCT | – | OCT |
| 245 | HEX | O | HEX | 266 | DEC | O | NON | 287 | OCT | – | NON |
| 246 | HEX | O | HEP | 267 | DEC | O | DEC | 288 | OCT | – | DEC |
| 247 | HEX | O | OCT | 268 | DEC | O | 2MB | 289 | NON | – | HEX |
| 248 | HEP | O | HEX | 269 | DEC | O | 4MH | 290 | NON | – | HEP |
| 249 | HEP | O | HEP | 270 | DEC | O | 2EPOr | 291 | NON | – | OCT |
| 250 | HEP | O | OCT | 271 | DEC | O | $CF_3$HEX | 292 | NON | – | NON |
| 251 | HEP | O | NON | 272 | PEN | – | MET | 293 | NON | – | DEC |
| 252 | HEP | O | DEC | 273 | PEN | – | ETY | 294 | DEC | – | HEX |
| 253 | OCT | O | HEX | 274 | PEN | – | PRO | 295 | DEC | – | HEP |
| 254 | OCT | O | HEP | 275 | HEX | – | BUT | 296 | DEC | – | OCT |
| 255 | OCT | O | OCT | 276 | HEX | – | HEX | 297 | DEC | – | NON |
| 256 | OCT | O | NON | 277 | HEX | – | HEP | 298 | DEC | – | DEC |
| 257 | OCT | O | DEC | 278 | HEX | – | OCT | 299 | DEC | – | 2MB |
| 258 | NON | O | HEX | 279 | HEP | – | HEX | 300 | DEC | – | 4MH |
| 259 | NON | O | HEP | 280 | HEP | – | HEP | 301 | DEC | – | 2EOPr |
| 260 | NON | O | OCT | 281 | HEP | – | OCT | 302 | DEC | – | $CF_3$HEX |
| 261 | NON | O | NON | 282 | HEP | – | NON | | | | |

Table 6. Formula (1) wherein A is Ph-$X^4$ and Y is -C≡C-

| No. | $R^1$ | X | $R^2$ | No. | $R^1$ | X | $R^2$ | No. | $R^1$ | X | $R^2$ |
|---|---|---|---|---|---|---|---|---|---|---|---|
| 303 | HEX | O | HEX | 312 | HEP | O | HEP | 321 | HEX | – | 4MH |
| 304 | HEX | O | HEP | 313 | HEP | O | OCT | 322 | HEX | – | 2EOPr |
| 305 | HEX | O | OCT | 314 | OCT | O | HEX | 323 | HEX | – | $CF_3$HEX |
| 306 | HEX | O | DEC | 315 | OCT | O | HEP | 324 | HEP | – | HEX |
| 307 | HEX | O | 2MB | 316 | OCT | O | OCT | 325 | HEP | – | HEP |
| 308 | HEX | O | 4MH | 317 | HEX | – | HEX | 326 | HEP | – | OCT |
| 309 | HEX | O | 2EOPr | 318 | HEX | – | HEP | 327 | OCT | – | HEX |
| 310 | HEX | O | $CF_3$HEX | 319 | HEX | – | OCT | 328 | OCT | – | HEP |
| 311 | HEP | O | HEX | 320 | HEX | – | 2MB | 329 | OCT | – | OCT |

Table 7. Formula (1) wherein A is Ph-$X^4$ and Y is $-CH_2CH_2-$

| No. | $R^1$ | X | $R^2$ | No. | $R^1$ | X | $R^2$ | No. | $R^1$ | X | $R^2$ |
|---|---|---|---|---|---|---|---|---|---|---|---|
| 330 | HEX | O | HEX | 339 | HEP | O | HEP | 348 | HEX | O | 4MH |
| 331 | HEX | O | HEP | 340 | HEP | O | OCT | 349 | HEX | O | 2EOPr |
| 332 | HEX | O | OCT | 341 | OCT | O | HEX | 350 | HEX | O | $CF_3$HEX |
| 333 | HEX | O | DEC | 342 | OCT | O | HEP | 351 | HEP | O | HEX |
| 334 | HEX | O | 2MB | 343 | OCT | O | OCT | 352 | HEP | O | HEP |
| 335 | HEX | O | 4MH | 344 | HEX | O | HEX | 353 | HEP | O | OCT |
| 336 | HEX | O | 2EOPr | 345 | HEX | O | HEP | 354 | OCT | O | HEX |
| 337 | HEX | O | $CF_3$HEX | 346 | HEX | O | OCT | 355 | OCT | O | HEP |
| 338 | HEP | O | HEX | 347 | HEX | O | 2MB | 356 | OCT | O | OCT |

Table 8. Formula (1) wherein A is $X^1$-Ph and Y is -C≡C-

| No. | $R^1$ | X | $R^2$ | No. | $R^1$ | X | $R^2$ | No. | $R^1$ | X | $R^2$ |
|---|---|---|---|---|---|---|---|---|---|---|---|
| 357 | HEX | O | HEX | 366 | HEP | O | OCT | 375 | HEX | – | 2EOPr |
| 358 | HEX | O | HEP | 367 | OCT | O | HEX | 376 | HEX | – | $CF_3$HEX |
| 359 | HEX | O | OCT | 368 | OCT | O | HEP | 377 | HEP | – | HEX |
| 360 | HEX | O | 2MB | 369 | OCT | O | OCT | 378 | HEP | – | HEP |
| 361 | HEX | O | 4MH | 370 | HEX | – | HEX | 379 | HEP | – | OCT |
| 362 | HEX | O | 2EOPr | 371 | HEX | – | HEP | 380 | OCT | – | HEX |
| 363 | HEX | O | $CF_3$HEX | 372 | HEX | – | OCT | 381 | OCT | – | HEP |
| 364 | HEP | O | HEX | 373 | HEX | – | 2MB | 382 | OCT | – | OCT |
| 365 | HEP | O | HEP | 374 | HEX | – | 4MH | | | | |

Table 9. Formula (1) wherein A is $X^1$-Ph and Y is $-CH_2CH_2-$

| No. | $R^1$ | X | $R^2$ | No. | $R^1$ | X | $R^2$ | No. | $R^1$ | X | $R^2$ |
|---|---|---|---|---|---|---|---|---|---|---|---|
| 383 | HEX | O | HEX | 392 | HEP | O | OCT | 401 | HEX | – | 2EOPr |
| 384 | HEX | O | HEP | 393 | OCT | O | HEX | 402 | HEX | – | $CF_3$HEX |
| 385 | HEX | O | OCT | 394 | OCT | O | HEP | 403 | HEP | – | HEX |
| 386 | HEX | O | 2MB | 395 | OCT | O | OCT | 404 | HEP | – | HEP |
| 387 | HEX | O | 4MH | 396 | HEX | – | HEX | 405 | HEP | – | OCT |
| 388 | HEX | O | 2EOPr | 397 | HEX | – | HEP | 406 | OCT | – | HEX |
| 389 | HEX | O | $CF_3$HEX | 398 | HEX | – | OCT | 407 | OCT | – | HEP |
| 390 | HEP | O | HEX | 399 | HEX | – | 2MB | 408 | OCT | – | OCT |
| 391 | HEP | O | HEP | 400 | HEX | – | 4MH | | | | |

Table 10. Formula (2) wherein $A^1$ is Ph, $A^2$ is $F_2$Ph, p is 1 and $R^2$ is $-(CH_2)_nC^*H(CH_3)R'$

| No. | $R^1$ | n | R' | No. | $R^1$ | n | R' | No. | $R^1$ | n | R' |
|---|---|---|---|---|---|---|---|---|---|---|---|
| 409 | DEC | 1 | ETY | 416 | NON | 5 | ETY | 423 | OCT | 1 | HEX |
| 410 | DEC | 3 | ETY | 417 | NON | 0 | HEX | 424 | UND | 1 | ETY |
| 411 | DEC | 5 | ETY | 418 | NON | 1 | HEX | 425 | UND | 3 | ETY |
| 412 | DEC | 0 | HEX | 419 | OCT | 1 | ETY | 426 | UND | 5 | ETY |
| 413 | DEC | 1 | HEX | 420 | OCT | 3 | ETY | 427 | UND | 0 | HEX |
| 414 | NON | 1 | ETY | 421 | OCT | 5 | ETY | 428 | UND | 1 | HEX |
| 415 | NON | 3 | ETY | 422 | OCT | 0 | HEX | | | | |

Table 11. Formula (2)

| No. | $R^1$ | $A^1$ | $A^2$ | p | $R^2$ | No. | $R^1$ | $A^1$ | $A^2$ | p | $R^2$ |
|---|---|---|---|---|---|---|---|---|---|---|---|
| 429 | NON | $F_2$Ph | Ph | 0 | NON | 435 | NON | Ph | $F_2$Ph | 1 | OCT |
| 430 | NON | Ph | $F_2$Ph | 0 | NON | 436 | NON | Ph | $F_2$Ph | 1 | HEP |
| 431 | DEC | $F_2$Ph | Ph | 0 | 4MH | 437 | NON | Ph | $F_2$Ph | 1 | NON |
| 432 | DEC | Ph | $F_2$Ph | 0 | 2MB | 438 | NON | $F_2$Ph | Ph | 1 | OCT |
| 433 | DEC | $F_2$Ph | Ph | 1 | 4MH | 439 | NON | $F_2$Ph | Ph | 1 | HEP |
| 434 | NON | Ph | $F_2$Ph | 1 | NON | | | | | | |

Compounds represented by the formula (1) can be produced as follows: in accordance with the following reactions [I] or [I'] in case where A is $X^4$ and Y is -C≡C-; in accordance with [II] in case where A is Ph and Y is -C≡C-; in accordance with [III] in case where A is $X^3$ and Y is -C≡C-; in accordance with [IV] in case where A is $X^2$ and Y is -C≡C-; in accordance with [V] in case where A is Ph-$X^4$ and Y is -C≡C-; in accordance with [VI] in case where A is Ph-$X^4$ and Y is -C≡C-; in accordance with [VII] in case where A is $X^1$-Ph and Y is -C≡C-; or in accordance with [VIII] in case where A is $X^1$-Ph and Y is $-CH_2-CH_2-$.

[I] Formula (1) wherein A is $X^4$ and Y is -C≡C-

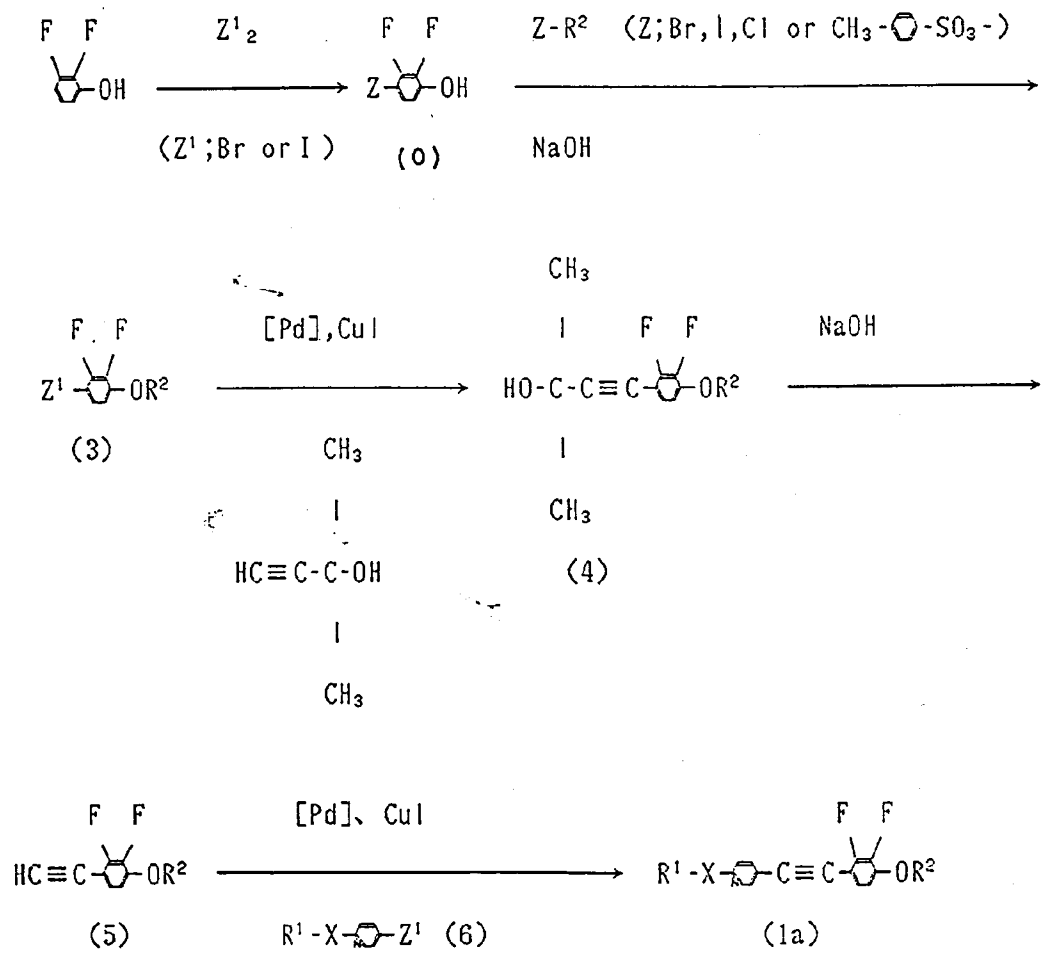


(0)

(3)

(4)

(5)

(6)

(1a)

A compound of the formula (0), prepared by reacting 2,3-difluorophenol with halogen on the ice bath, is reacted with an alkylating reagent (such as alkyl halide) in the presence of sodium hydroxide within dimethylsulfoxide (hereinafter referred to as DMSO) to obtain a compound of the formula (3). The compound of the formula (3) is reacted with 3-methyl-1-butyne-3-ol in triethylamine under an atmosphere of inert gas in the presence of zero-or bi-valent palladium catalyst to obtain a compound of the formula (1a), within the scope of this invention.

[I'] Formula (1) wherein A is $X^4$ and Y is -C≡C-

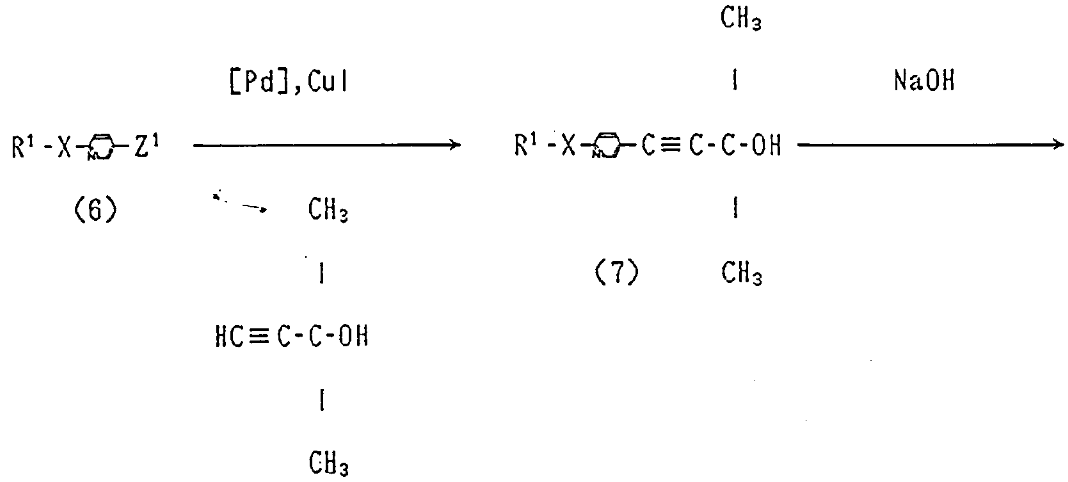


(6) (7)

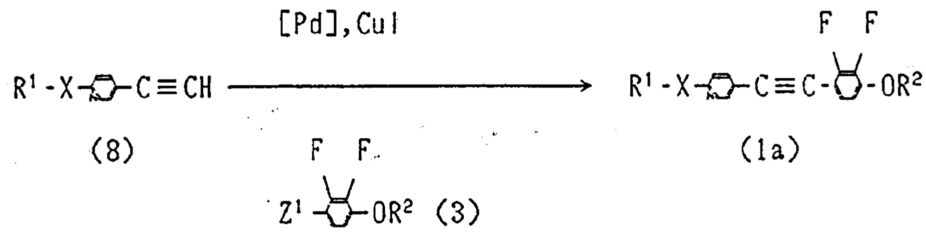


(8) (3) (1a)

A compound of the formula (6) is reacted with 3-methyl-1-butyne-3-ol in triethylamine under an atmosphere of inert gas in the presence of zero- or bi-valent palladium catalyst to obtain a compound of the formula (7), followed by reacting this compound with sodium hydroxide powder in anhydrous toluene to obtain a compound of the formula (8). This compound and the compound of the formula (3) are reacted in triethylamine under an atmosphere of inert gas in the presence of zero- or bi-valent palladium catalyst to obtain a compound of the formula (1a) of the present invention.

The compound of the formula (6) as a raw material can be produced as follows: in accordance with [I-1] in case where X is direct bond; and in accordance with [I-2] in case where X is O.

[I-1] In case of X=direct bond

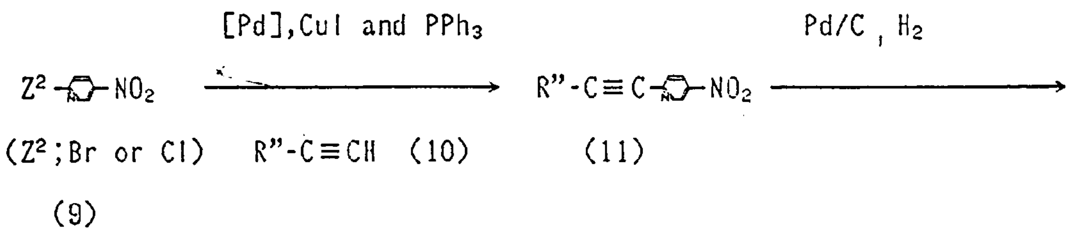


(9) (10) (11)

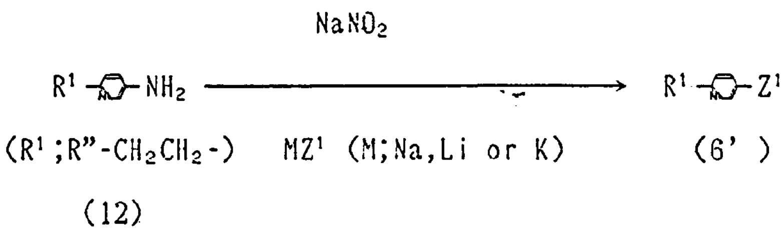


(12) (6')

A compound of the formula (9) and a compound of the formula (10) are reacted in triethylamine under an atmosphere of inert gas in the presence of zero- or bi-valent palladium catalyst to obtain a compound of the formula (11), followed by hydrogenating and reducing this compound in ethanol in the presence of 5% palladium-carbon as catalyst to obtain a compound of the formula (12). This compound is diazotized with sodium nitrite, followed by reacting an alkali metal iodide to obtain a compound of the formula (6′).

[I-2] In case of X=O

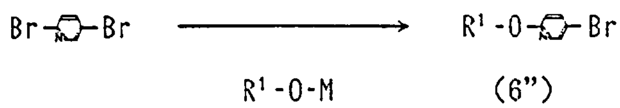


(6")

An alkali metal alcholate is reduced with 2,5-dibromopyridyne in anhydrous dimethylformamide (hereinafter referred to as DMF) to obtain a compound of the formula (6″).

[I-3] In case of X=C≡C

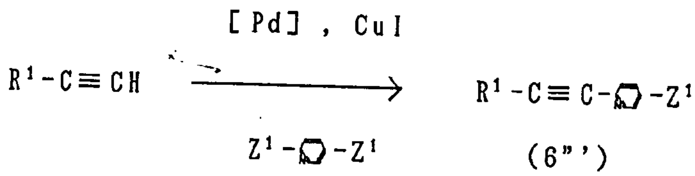


(6"')

An 1-alkyne (such as 1-decyne) is reduced with 2,5-dihalogenoopyridyne (such as 2,5-dibromopyridyne) in triethylamine under an atmosphere of inert gas in the presence of zero- or bi-valent palladium catalyst to obtain a compound of the formula (6‴).

[II] Formula (1) wherein A is Ph and Y is -C≡C-

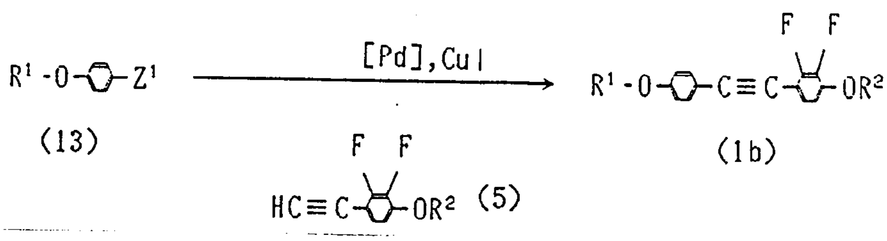


(13) (5) (1b)

A compound of the formula (13) and a compound of the formula (5) are reacted in triethylamine under an atmosphere of inert gas in the presence of zero- or bi-valent palladium catalyst to obtain a compound of the formula (1b), within the scope of the present invention.

[III] Formula (1) wherein A is $X^3$, Y is -C≡C-

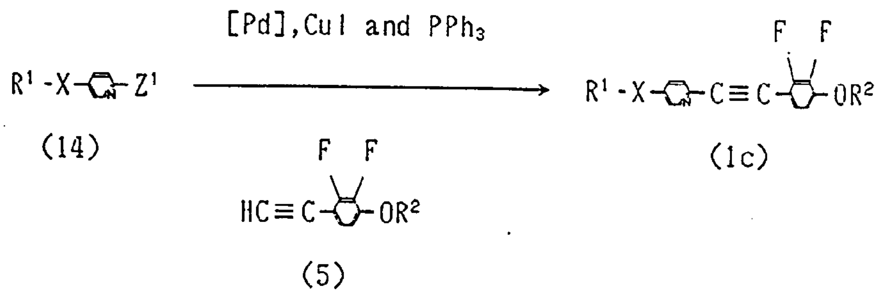


(14) (1c) (5)

A compound of the formula (14) and a compound of the formula (5) are reacted in triethylamine under an atmosphere of inert gas in the presence of zero- or bivalent palladium catalyst to obtain a compound of the formula (1b), within the scope of the present invention.

The compound of the formula (14) as a raw material can be produced as follows: in accordance with [III-1] in case where X is direct bond; and in accordance with [III-2] in case where X is O.

[III-1] In case of X=direct bond

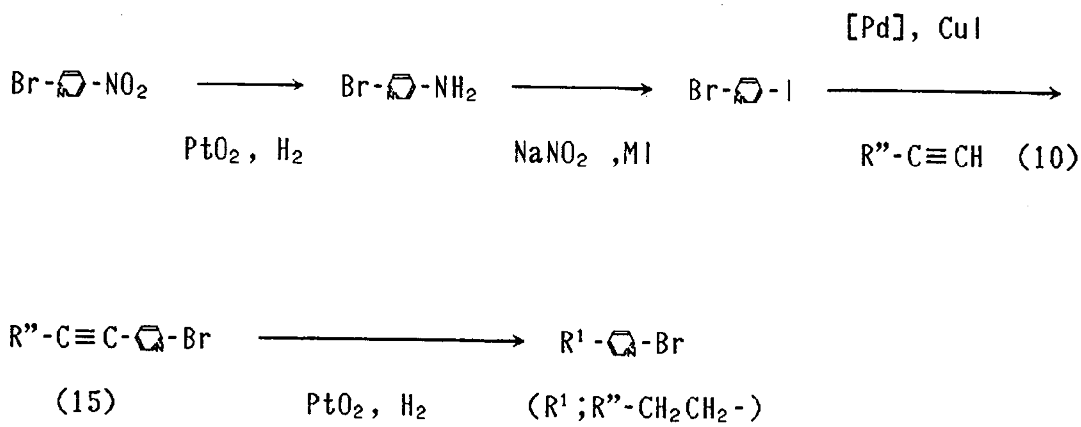


(15) (14')

2-bromo-5-aminopyridine, obtained by hydrogenating 2-bromo-5-nitropyridine in ethanol in the presence of platinum dioxide as catalyst, is diazotized with sodium nitrite, followed by reacting an alkali metal iodide to obtain 2-bromo-5-iodopyridine. 2-bromo-5-iodopyridine is reacted with a compound of the formula (10) in triethylamine under an atmosphere of inert gas in the presence of zero- or bi-valent palladium catalyst to obtain a compound of the formula (15), followed by hydrogenating this compound in ethanol in the presence of platinum dioxide as catalyst to obtain a compound of the formula (14′).

[III-2] In case of X=O

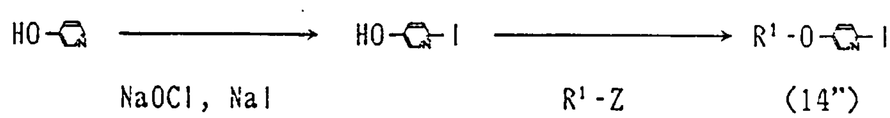


(14")

2-iodo-5-hydroxypyridine, obtained by adding sodium iodide and aqueous solution of sodium hypochlorite to 3-hydroxypyridine under ice bath, is reacted with an alkylating agent (such as alkyl halides) to obtain a compound of the formula (14″).

[IV] Formula (1) wherein A is $X^2$ and Y is -C≡C-

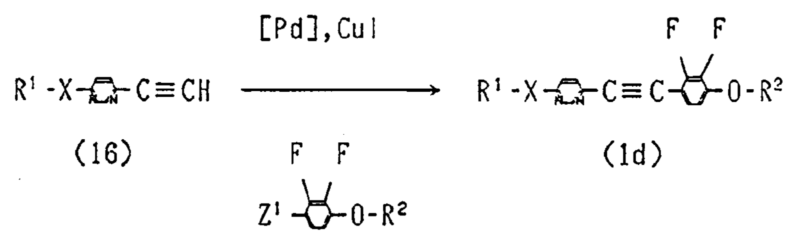


(16) (1d) (3)

A compound of the formula (16) and a compound of the formula (3) are reacted in triethylamine under an atmosphere of inert gas in the presence of zero- or bi-valent palladium catalyst to obtain a compound of the

formula (1d), within the scope of the present invention.

The compound of the formula (16) as a raw material can be produced as follows: in accordance with [IV-1] in case where X is direct bond; and in accordance with [IV-2] in case where X is O.

[IV-1] In case of X=direct bond

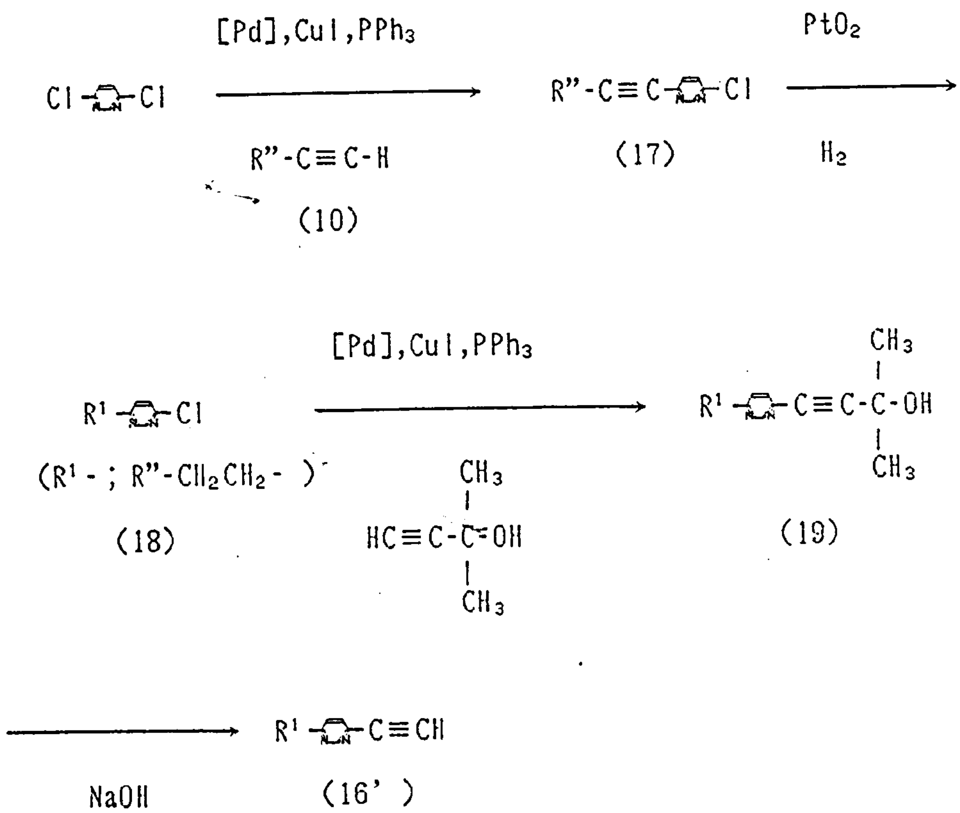

(17)
(10)
(18)
(19)
(16')

3,6-dichloropyridazine is reacted with a compound of the formula (10) in triethylamine under an atmosphere of inert gas in the presence of zero- or bi-valent palladium catalyst to obtain a compound of the formula (17), followed by hydrogenating this compound in ethanol in the presence of 5% palladium carbon catalyst to obtain a compound of the formula (18). This compound and 3-methylbutyne-3-ol are reacted in triethylamine under an atmosphere of inert gas in the presence of zero- or bi-valent palladium catalyst to obtain a compound of the formula (19), followed by reacting this compound with sodium hydroxide powder in anhydrous toluene to obtain a compound of the formula (16').

[IV-2] In case of X=o

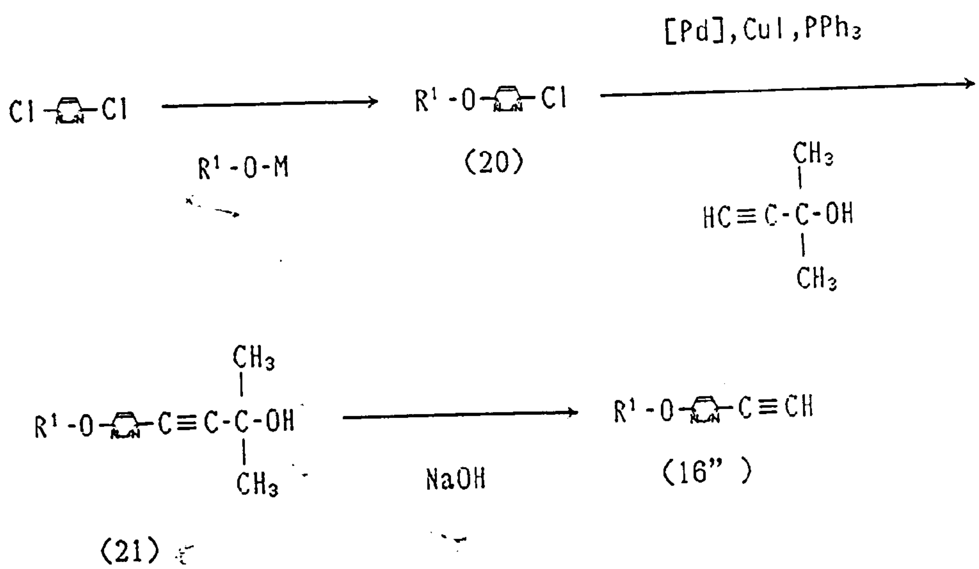


A compound of the formula (20), obtained by reacting an alkali metal alcholate with 3,6-dichloropyridazine in anhydrous DMF, is reacted with 3-methyl-1-butyne-3-ol in triethylamine under an atmosphere of inert gas in the presence of zero- or bi-valent palladium catalyst to obtain a compound of the formula (21), followed by reacting this compound with sodium hydroxide powder in anhydrous toluene to obtain a compound of the formula (16″).

[V] Formula (1) wherein A is -Ph-X⁴-, Y is -C≡C-

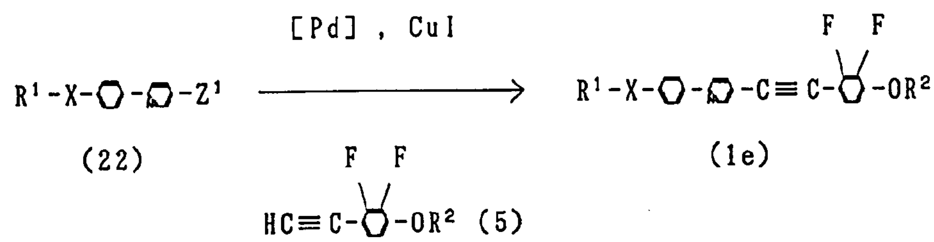


A compound of the formula (22) and a compound of the formula (5) are reacted in triethylamine under an atmosphere of inert gas in the presence of zero- or bi-valent palladium catalyst to obtain a compound of the formula (1e), within the scope of the present invention.

The compound of the formula (22) as a raw material can be produced as follows.

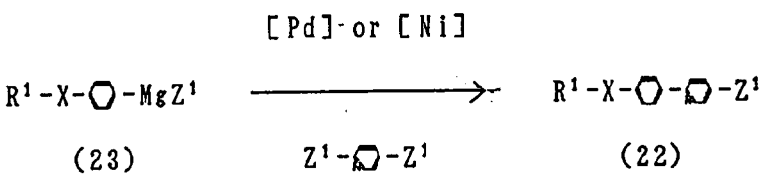


A compound of the formula (23) is reacted with 2,5-dihalogenopyridine, such as 2,5-dibromopyridine, under an atmosphere of inert gas in the presence of zero- or bi-valent palladiun or nickel catalyst to obtain a compound of the formula (22).

The compound of the formula (23) can be produced as follows: in accordance with [V-1] in case where X is direct bond; and in accordance with [V-2] in case where X is 0.

[V-1] In case of X is direct bond

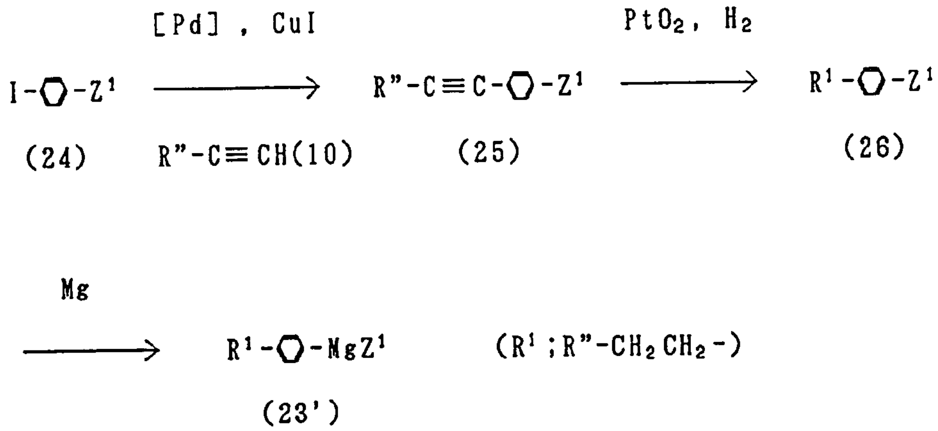

(24) (25) (26) (23')

A compound of the formula (24) and a compound of the formula (10) are reacted in triethylamine under an atmosphere of inert gas in the presence of zero- or bi-valent palladium catalyst, followed by hydrogenating this compound in ethanol in the presence of platinum dioxide as catalyst to obtain a compound of the formula (26). This compound is reacted with magnesium metal to obtain a compound of the formula (23′).

[V-2] In case of X is 0

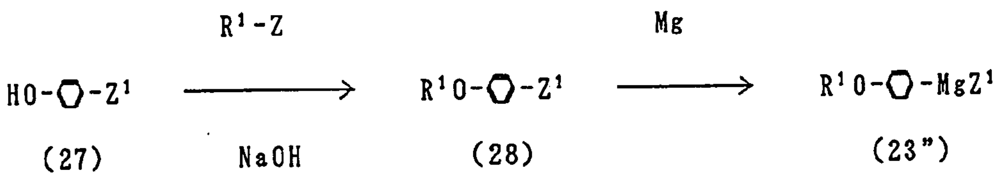

(27) (28) (23")

A compound of the formula (27) is reacted with a halogenating reagent (such as alkyl halide) in the presence of sodium hydroxide within DMSO to obtain a compound of the formula (28). This compound is reacted with magnesium metal to obtain a compound of the formula (23″).

[VI] Formula (1) wherein A is -Ph-$X^4$-, Y is -$CH_2CH_2$-

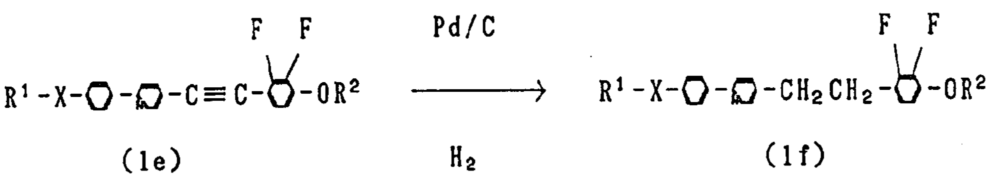

(1e) (1f)

A compound of the formula (1e), obtaied [V] , is hydrogenated in ethanol in the presence of 5% palladium carbon as catalyst to obtain a compound of the formula (1f), within the scope of the present invention.

[VII] Firmula (1) wherein A is -$X^1$-Ph-, Y is -C≡C-

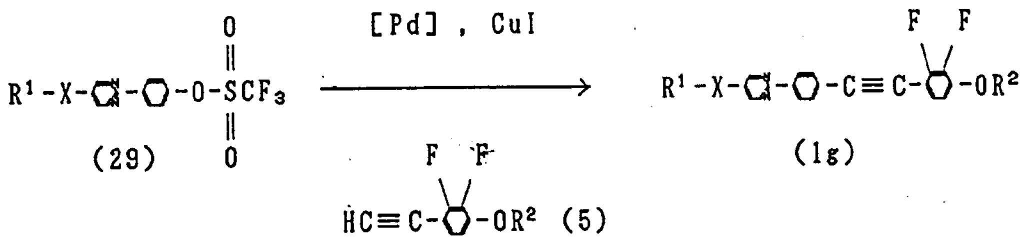


(29) (5) (1g)

A compound of the formula (29) and a compound of the formula (5) are reacted in triethylamine under an atmosphere of inert gas in the presence of zero- or bi-valent palladium catalyst to obtain a compound of the formula (1g), within the scope of the present invention.

The compound of the formula (29) as a raw material can be produced as follows.

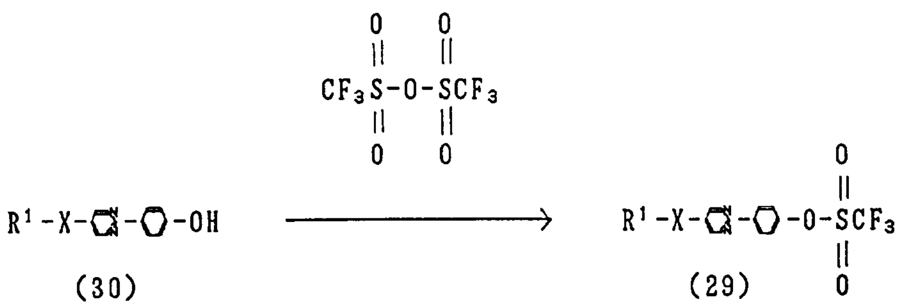

(30) (29)

A compound of the formula (30) is reacted with trifluoromethanesulufonic anhydride in pyridine to obtain a compound of the formula (29).

[VIII] Formula (1) wherein A is -$X^1$-Ph-, Y is -$CH_2CH_2$-

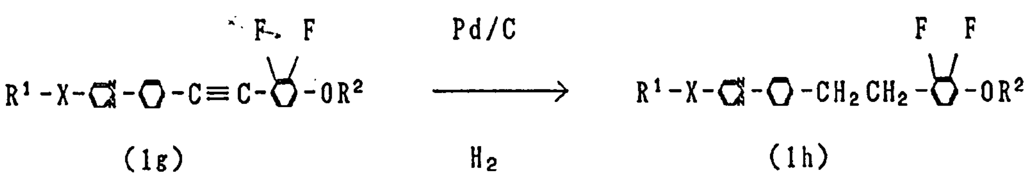


(1g) (1h)

A compound of the formula (1g), obtaied [VII] , is hydrogenated in ethanol in the presence of 5% palladium carbon as catalyst to obtain a compound of the formula (1h), within the scope of the present invention.

Compounds represented by the formula (2) can be produced in accordance with the following reactions [IX], [X], [XI], [XII] or [XIII].

[IX] Formula (2) wherein $A^1$ is $F_2Ph$, $A^2$ is Ph, p is 1

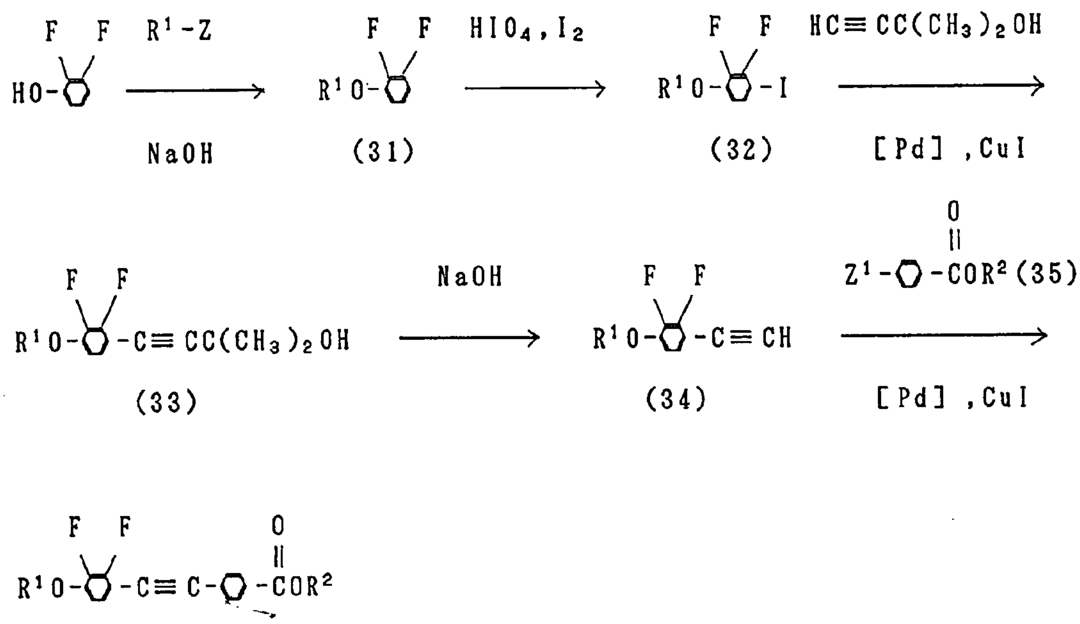


(31) (32) (33) (34)

(2a)

2,3-difluorophenol and alkylating reagent (such as alkyl halides) are reacted in the presence of sodium hydroxide to obtain a compound of the formula (31), followed by reacting this compound with periodic acid and iodine to obtain a compound of the formula (32). This compound is reacted with 3-metyl-1-butyn-3-ol in triethylamine under an atmosphere of inert gas in the presence of zero- or bi-valent palladium catalyst to obtain a compound of the formula (33), followed by reacting this compound with sodium hydroxide powder in anhydrous toluene to obtain a compound of the formula (34). This compound and the compound of the formula (35) are reacted in triethylamine under an atmosphere of inert gas in the presence of zero- or bi-valent palladium catalyst to obtain a compound of the formula (2a) of the present invention.

The compound of the formula (35) can be produced as follows

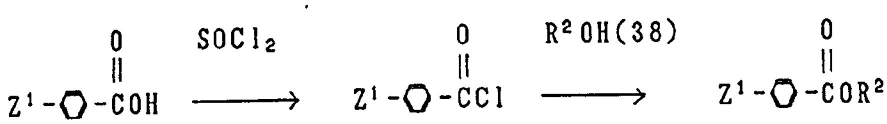


(36) (37) (35)

A compound of the formula (37), prepared from a compound of the formula (36) and thionyl chloride, is reacted with a compound of the formula (38) in the presence of amine (such as pyridine) to obtain a compound of the formula (35).

[X] Formula (2) wherein $A^1$ is $F_2Ph$, $A^2$ is Ph, p is 0

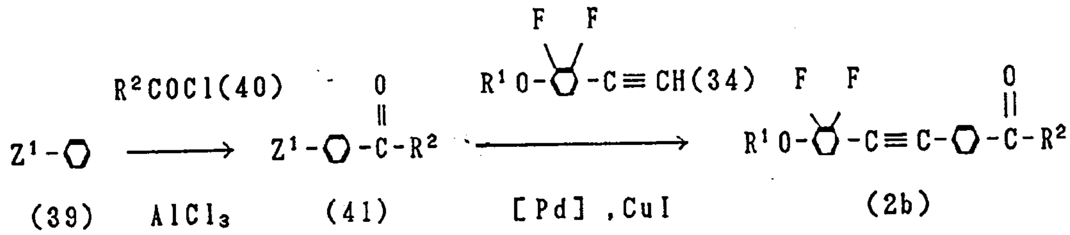


A compound of the formula (39) and a compound of the formula (40) are reacted in the presence of alminium trichloride to obtain a compound of the formula (41). This compound and the compound of the formula (34) are reacted in triethylanine under an atmosphere of inert gas in the presence of zero- or bi-valent palladium catalyst to obtain a compound of the formula (2b) of the present invention.

[XI] Formula (2) wherein $A^1$ is $F_2Ph$, $A^2$ is Ph, p is 0

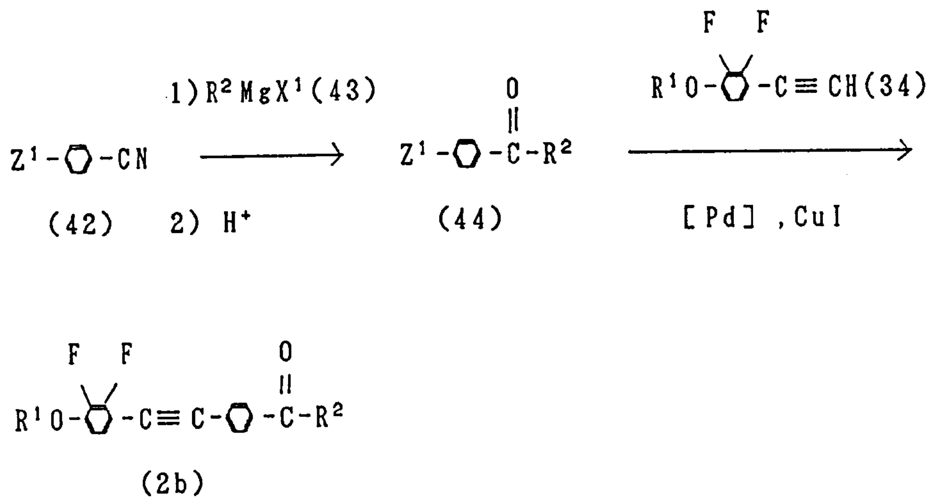


A compound of the formula (42) and a compound of the formula (43) are reacted in diethyl ether/benzen at reflux temperature, followed by adding 6N hydrochloric acid to obtain a compound of the formula (44). This compound and the compound of the formula (34) are reacted in triethylamine under an atmosphere of inert gas in the presence of zero- or bi-valent palladium catalyst to obtain a compound of the formula (2b) of the present invention.

[XII] Formula (2) wherein $A^1$ is Ph, $A^2$ is $F_2Ph$, p is 0

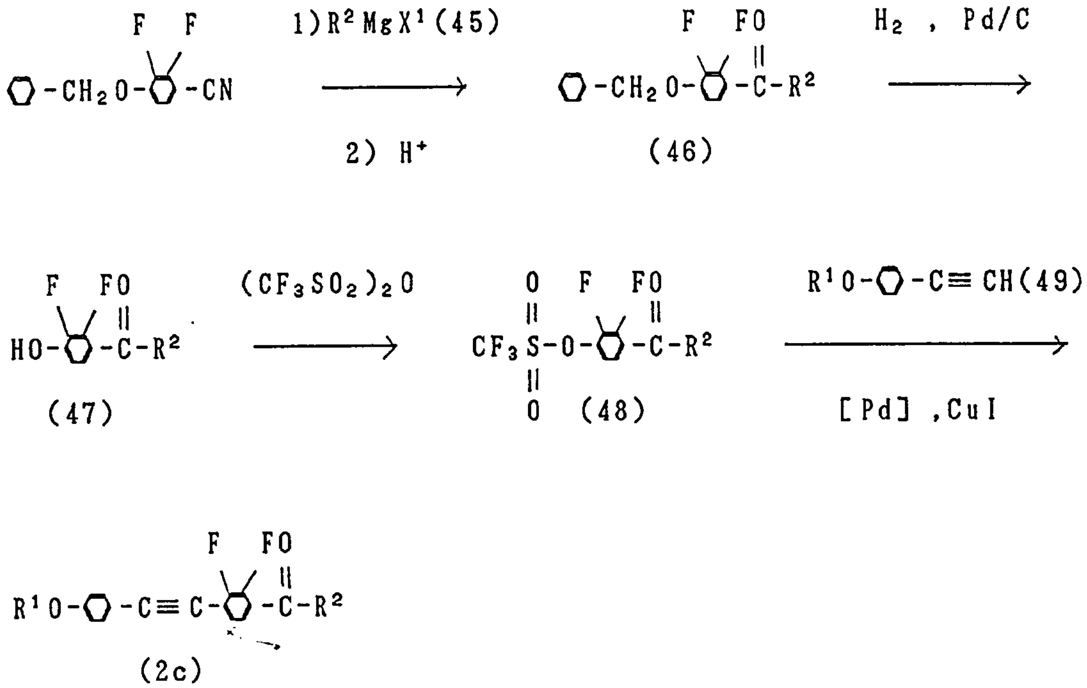


A 1-cyano-2,3-difluoro-4-benzyloxybenzene and a compound of the formula (45) are reacted in diethyl ether/benzene at reflux temperature, followed by adding 6N hydrochloric acid to obtain a compound of the formula (46), followed by hydrogenating this compound in ethanol in the presence of 5% palladium-carbon as catalyst to obtain a compound of the formula (47). This compound is reacted with trifluoromethanesulufonic anhydride in pyridine to obtain a compound of the formula (48). This compound and the compound of the formula (49) are reacted in triethylanine under an atmosphere of inert gas in the presence of zero- or bi-valent palladium catalyst to obtain a compound of the formula (2c) of the present invention.

The 1-cyano-2,3-difluoro-4-benzyloxybenzene can be produced as follows.

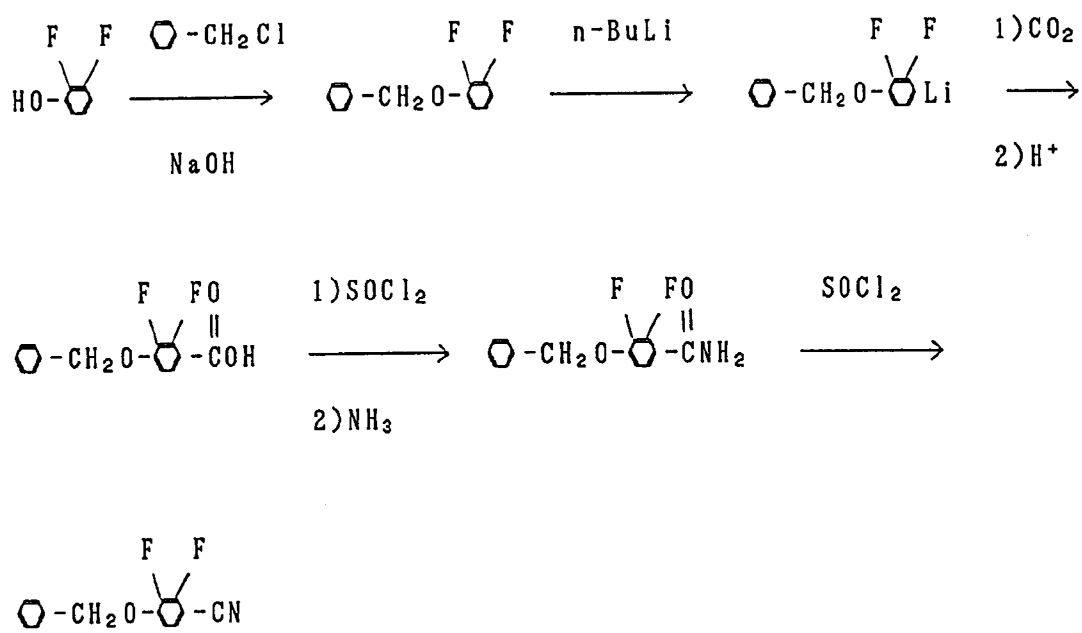

A 2,3-difluorophenol is reacted with benzyl chloride to obtain a 2,3-difluoro-4-benzyloxybenzene. This compound is lithiated by n-buthyl lithium, followed by reacting with carbon dioxide to obtain a 2,3-difluoro-4-benzyloxybenzoic acid. The 2,3-difluoro-4-benzyloxybenzoic acid is reacted with thionyl chloride, followed by reacting with ammonia to obtain a 2,3-difluoro-4-benzyloxybenzamide. This compound is dehydrated by thionyl chloride to obtain a 2,3-difluoro-4-benzyloxycyanobenzene.

[XIII] Formula (2) wherein $A^1$ is Ph, $A^2$ is $F_2Ph$, p is 1 and $R^2$ is $-(CH_2)_nC^*H(CH_3)R'$

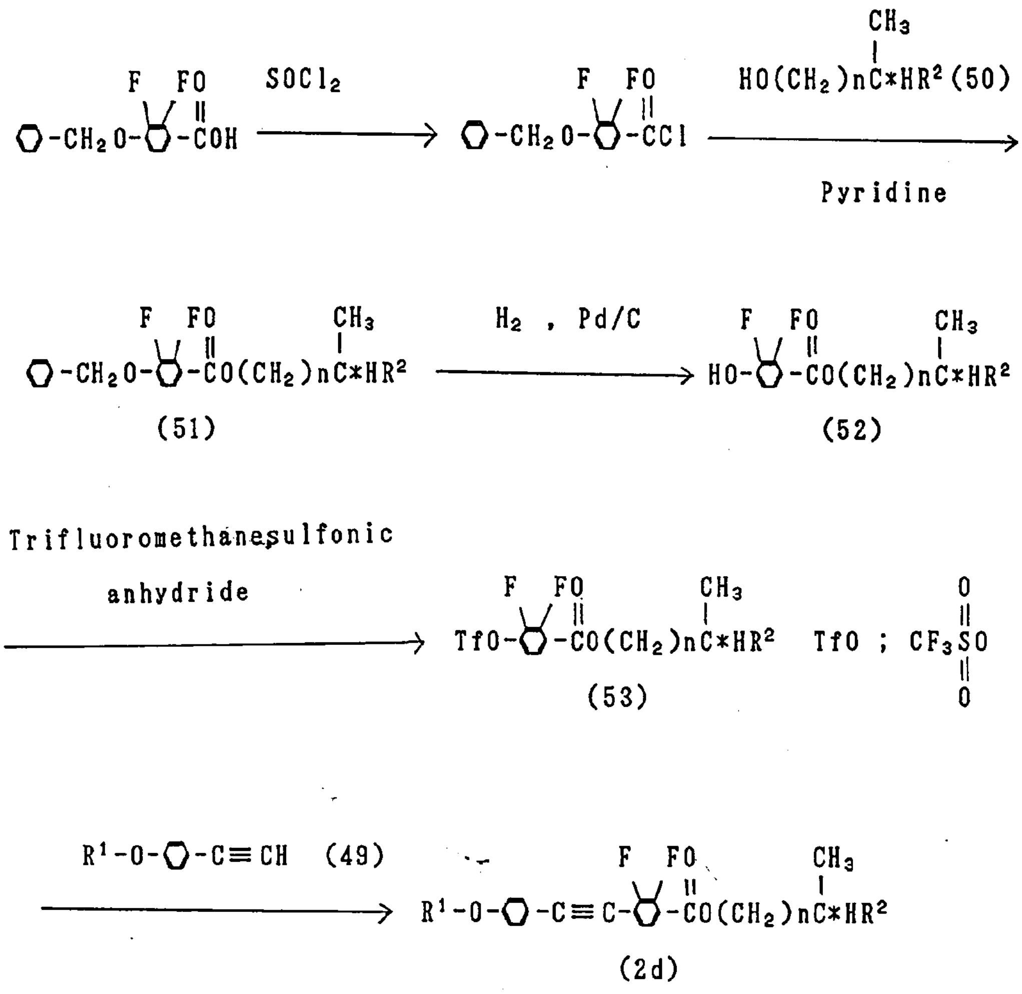

(50)
(51)
(52)
(53)
(49)
(2d)

By reaction with thionyl chloride, 4-benzyloxy-2,3-difluorobenzoic acid is converted into the corresponding acid chloride, and then reacted with an alcohol of the formula (50) in the presence of a base (such as pyridine) to obtain a compound of the formula (51). This compound is hydrogenolyzed with palladium carbon and then reacted with trifluoromethane sulfonic anhydride to obtain a compound of the formula (53). This compound (53) and a compound of the formula (49) are reacted within an atmosphere of an inert gas in the presence of palladium catalyst to produce a compound of the formula (2d), within the scope of the present invention. In case where the alcohol (50) is optically active one, the resulting liquid crystal compound (2d) shows chiral smectic C phase; and when the alcohol (50) is racemic one, the resulting liquid crystal compound (2d) shows smectic C phase.

The raw material 4-benzyloxy-2,3-difluorobenzoic acid can be produced as follows:

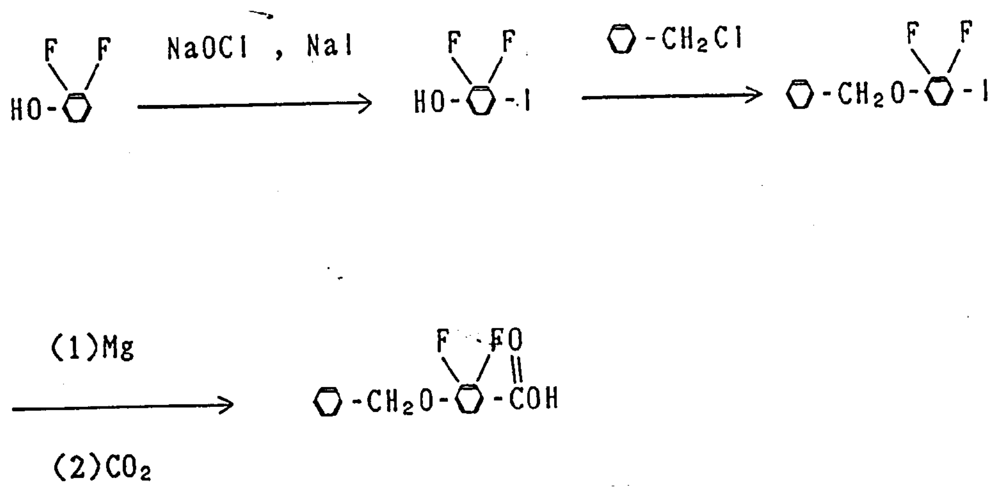


That is, 2,3-difluorophenol is iodided with sodium hypochlorite and sodium iodide, etherified with benzyl chloride, and then reacted with metallic magnesium into a Grignard's compound, follwed by reacting the compound with carbon dioxide to obtain 4-benzyloxy-2,3-difluorobenzoic acid.

Liquid crystal compounds of the present invention containing optically active $R^1$ and/or $R^2$ group show chiral smectic C phase, and liquid crystal compounds of this invention containing $R^1$ and/or $R^2$ group free from optical activity show smectic C phase. In general, liquid crystals are used in the forms of liquid crystal compositions containing two or more components; and compounds of the present invention can be used as a component of liquid crystal compositions. Liquid crystal composition according to the present invention comprises a mixture of a pluralty of compounds, comprising at least one compound of this invention. Liquid crystal compositions of the invention include, for example, [1] ones showing smectic C phase, and [2] ones showing chiral smectic C phase.

Liquid crystal compositions [1] of the present invention comprise at least one liquid crystal compound of this invention showing smectic C phase as the essential component, and can optionally contain one or more other liquid crystal compounds, which may be selected from the group consisting of other liquid crystal compounds showing smectic C phase, liquid crystal compounds showing smectic phase other than smectic C or chiral smectic C phase, and liquid crystal compounds showing nematic phase. These liquid crystal compounds include, for example, those written in EP 220,296. Illustrative of suitable liquid crystal compounds showing smectic C phase are 2-4′-alkoxyphenyl-5-alkylpyrimidine, 2-4′-alkylphenyl-5-alkoxypyrimidine, 2-4′-alkoxyphenyl-5-alkoxypyrimidine, 2-4′-alkylphenyl-5-alkylpyrimidine, 2-p-alkoxycarbonylphenyl-5-alkylprimidine, 2-4′-alkoxy-3′-fluorophenyl-5-alkylpyrimidine, 2-4′-alkoxy-2′,3′-difluoro-phenyl-5-alkylpyrimidine, 2-4′-alkoxyphenyl-5-alkylpyridine, 2-4′-alkoxy-3′-fluorophenyl-5-alkylpyridine, 2-4′-alkoxy-2′,3′-difluorophenyl-5-alkylpyridine, 2-4′-alkylbiphenyl-5-alkylpyrimidine, 2-4′-alkoxybiphenyl-5-alkylpyrimidine, 2-4′-alkoxybiphenyl-5-alkoxypyrimidine, 2-4′-alkylbiphenyl-5-alkylpyrimidine, 2-4′-alkylphenyl-5-4′-alkoxyphenylpyrimidine, 2-4′-alkoxyphenyl-5-4′-alkoxyphenylpyrimidine, 2-4′-alkoxyphenyl-5-4′-alkylphenylpyrimidine, 2-4′-alkylphenyl-5-4′-alkoxyphenylpyrimidine, 1-5′-alkyl-2′-pyridyl-2-4′-alkoxyphenylacetylene, 1-2′-alkyl-5′-pyridyl-2-4′-alkoxyphenylacetylene, 1-2′-alkyl-5′-pyridyl-2-4′-alkoxy-3′-fluorophenylacetylene, 1-3′-alkyl-5′-pyridazyl-2-4′-alkoxy-3′-fluorophenylacetylene, and 1-3′-alkoxy-5′-pyridazyl-2-4′-alkoxy-3′-fluorophenylacetylene.

Liquid crystal compositions [2] of the present invention comprise at least one liquid crystal compound of this invention as the essential component and optionally one or more other liquid crystal compounds, and contain at least one optically active liquid crystal compound which may be the compound of this invention or/and the other liquid crystal compound. Liquid crystal compositions [2] include, for example, ones comprising said liquid crystal composition [1] and at least one optically active compound selected from the group consisting of compounds of this invention showing chiral smectic C phase and other liquid crystal compounds showing chiral smectic C phase. Illustrative examples of suitable other liquid crystal compounds include ones showing chiral smectic C phase, for example oa p′-(2-methylbutyloxycarbonyl)phenyl 4-alkoxy-4′-biphenylcarboxylate, oa 2-methylbutyl 4-alkoxy-4′-biphenylcarboxylate; oa 2-(2-methylbutyloxycarbonyl)naphthalene-6-4′-n-alkoxyphenylcarboxylate, oa 2-(1-methylheptyloxycarbonyl)naphthalene-6-4′-n-alkoxyphenylcarboxylate, oa 2-(2-methylbutyloxy)naphthalene-6-4′-n-alkoxyphenylcarboxylate and oa 2-(1-methylheptyloxy)-naphthalene-6-4′-n-alkoxyphenylcarboxylate; and other optically active compounds, for instance, those written in JPN Patent Lay-open Nos. 99032/1988, 190843/1988, 138274/1990 and 27374/1991, and EP Patents 374,789, 365,820 and 395,078 (corresponding to JPN Patent Lay-open Nos.256673/1990, 262579/1990 and 286673/1990, respectively). Illustrative of these compounds are oa 2-(1-cyanoethyl)-naphthalene-6-4′-n-alkylphenylcarboxylate, 2-(1-cyanoethyl)-naphthalene-6-4′-n-alkoxyphenylcarboxylate, oa 4-(1-trifluoromethylheptyloxymethyl)-4′-n-alkoxybiphenyl, oa 4-(1-trifluoromethylheptyloxymethyl)-4′-n-alkylbiphenyl, oa trans 4-(4′-n-alkoxyphenyl)-1-(1′-trifluoromethylheptyloxycarbonyl)cyclohexane, and the like.

Liquid crystal compositions may contain other components, for example, chiral compounds showing no liquid crystaline properties, pleochroic dyes (such as anthraquinone dyes, azo dyes and the like), and so on.

Content of said liquid crystal compound of this invention in liquid crystal compositions of the invention, which is not particularly restricted and can be varied widely, is usually 5-100 % by weight (5-99% in case of compositions [2]). Content of pleochroic dye is usually 0-5 % by weight.

By applied voltage, ferroelectric liquid crystal compositions of this invention cause photo-switching phenomena, which are applied for producing display elements of quick response [See for example, JPN Patent Lay-open Nos.107216/1981 and 118744/1984, and N.A.Clark and S.T.Lagerwall:Applied Physics Letters,36,899(1980)].

Liquid crystal compositions, showing ferroelectricity, according to the invention, bring about optical switching phenomena by applied voltage, and can provide display devices, such as those described in JPN Lay-open Patents No.107216/1981 and No.118744/1984, and N.A.Clark and S.T.Lagerwall, "Applied Physics Letters" 36,899(1980).

The liquid crystal compositions of this invention can be used as photo-switching elements (display devices), for instance, by sealing in vacuum into liquid crystal cells having cell distance of 0.5-10 (preferably 0.5-3) micron m, followed by providing polarizers on both sides.

The above liquid crystal cells can be produced by combining, through a spacer, two surface-aligned glass substrates provided with transparent electrodes. Examples of suitable spacers are alumina beads, glass fiber and polyimide film. Alignment can be done by conventional alignment treatment, such as application of polyimide membrane, rubbing treatment, oblique vapor-deposition of SiO.

Having generally described the invention, a more complete understanding can be obtained by reference to certain specific examples, which are included for purposes of illustration only and not intended to be limiting unless otherwise specified. In the following Examples, % represents % by weight.

Example 1 [Compound No.70]

1) To 45.00 g of 2,3-difluorophenol dissolved in 250 ml of chloroform, 55.40 g of bromine dissolved in chloroform were added dropwise under cooling with ice bath, and stirred for a while at room temperature, followed by washing with water and then distilling off chloroform. Thereafter, the product was recrystalized with hexane to obtain 14.30 g of 2,3-difluoro-4-bromophenol.

2) To 700 g of 2,3-difluoro-4-bromophenol dissolved in 50 ml DMSO, were added 1.47 g of sodium hydroxide and 6.34 g of n-octylbromide, and stirred for 7 days at room temperature. The resulting reaction product was extracted with hexane and then washed with water, followed by distilling off hexane to obtain 9.45 g of oily 2,3-difluoro-4-bromooctyloxybenzene.

3) In 50 ml of triethylamine, 1.90 g of 2,3-difluoro-4-bromooctyloxybenzene and 0.60 g of 3-methyl-1-butyne-3-ol were reacted in the presence of 30 mg of dichlorobistriphenylphosphine palladium$^{II}$, 8 mg of cuprous iodide and 60 mg of triphenylphosphine, within an atmosphere of nitrogen, under reflux for 12 hours. After reaction, triethylamine was removed, and the product was extracted with hexane. The hexane-containing layer was washed with 1 N-hydrochloric acid and then with water, followed by distilling off hexane under reduced pressure to obtain 1.79 g of an oily compound (a) represented by the formula:

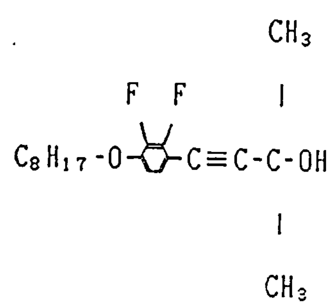

(a)

4) Into 100 ml of unhydrous toluene, were dissolved 1.79 g of the compound (a), followed by adding thereto 0.47 g of sodium hydroxide powder and then heating under reflux for an hour. After reaction, toluene was removed, and the product was extracted with ether and washed with water, followed by distilling off ether

under reduced pressure to obtain 1.16 g of an oily compound (b) represented by the formula:

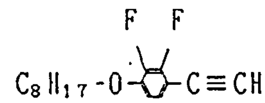

(b)

5) In 50 ml of triethylamine, 5.00 g of 2-bromo-5-nitropyridine and 3.50 g of 1-octyne were reacted in the presence of 204 mg of dichlorobistriphenylphosphine palladium$^{II}$ and 51 mg of cuprous iodide, within an atmosphere of nitrogen, at room temperature for 24 hours. After reaction, triethylamine was removed, and the product was extracted with hexane. The hexane-containing layer was washed with 1 N-hydrochloric acid and then with water. Thereafter, the product was dissolved in toluene, treated with silica gel column, and then recrystalized with methanol to obtain 3.00 g of a compound (c) represented by the formula:

$C_6H_{13}$-C≡C-$X^4$-$NO_2$ (c)

6) To 30 ml of ethanol containing 3.00 g of the compound (c), were added 0.50 g of 5% palladium-carbon as catalyst, and hydrogenation and reduction were carried out at room temperature under normal pressure within an atmosphere of hydrogen. After confirming that no absorption of hydrogen was observed, the catalyst was filterred off, followed by distilling off ethanol under reduced pressure to obtain 2.60 g of oily 2-n-octyl-5-aminopyridine.

7) Into 100 ml of water containing 11.00 g of 36% hydrochloric acid, were added 2.60 g of 2-n-octyl-5-aminopyridine, and cooled to a temperature below 5°C. A solution of 0.72 g of sodium nitrite in 5 ml of water was added thereto dropwise at a temperature below 5°C, followed by continuing stirring for additional 1 hour. Then a solution of 8.60 g of potassium iodide in 10 ml of water was added thereto, and the temperature restored gradually to room temperature, followed by continuing stirring until no generation of nitrogen was observed. Thereafter, the solution was alkalized with sodium hydroxide and extracted with hexane. The hexane-containing layer was washed with water, then with an aqueous sodium hydrogen sulfite and again with water, followed by treating with silica gel column to obtain 2.40 g of oily 2-n-octyl-5-iodopyridine.

8) In 50 ml of triethylamine, 1.32 g of 2-octyl-5-iodopyridine and 1.11 g of the compound (b) were reacted in the presence of 15 mg of dichlorobistriphenylphosphine palladium$^{II}$ and 4 mg of cuprous iodide, within an atmosphere of nitrogen, at room temperature for 4 hours. After reaction, triethylamine was removed, and the product was extracted with toluene. The resulting product was washed with 1 N-hydrochloric acid and then with water, and was purified with silica gel column, followed by recrystalizing four times with methanol to obtain 0.41 g of white crystal of Compound No.70 in Table 2, within the scope of the present invention.

IR spectrum, H-NMR spectrum and F-NMR spectrum of this compound were as shown in Fig.1(a), Fig.1(b) and Fig.1(c), respectively.

| | | | | | |
|---|---|---|---|---|---|
| Elemental | Observed | C:76.53 | H:8.58 | N:3.01 | F:8.30 |
| analysis,% | Theoretical | C:76.48 | H:8.57 | N:3.08 | F:8.35 |

Example 2 [Compound No.76]

1) Into 50 ml of DMSO, were dissolved 9.30 g of 2,3-difluoro-4-bromophenol as prepared in 1) of Example 1; and 1.96 g of sodium hydroxide and 9.03 g of n-nonylbromide were added and stirred for 7 days at room temperature. The resulting reaction product was extracted with hexane and then washed with water, followed by distilling off hexane to obtain 13.27 g of oily 2,3-difluoro-4-bromo-n-nonyloxybenzene.

2) In the same manner as in 3) and 4) of Example 1, 7.00 g of 2,3-difluoro-4-bromo-n-nonyloxybenzene were reacted to obtain 4.18 g of a compound (d) represented by the formula:

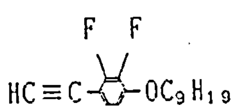

(d)

3) In the same manner as in 5), 6) and 7) of Example 1 except that 1-nonyne was used instead of 1-octyne, were obtained 3.50 g of 2-n-nonyl-5-iodopyridine.

4) In 50 ml of triethylamine, 2.47 g of 2-n-nonyl-5-iodopyridine and 2.09 g of the compound (d) were reacted in the presence of 26 mg of dichlorobistriphenylphosphine palladium$^{II}$ and 7 mg of cuprous iodide, within an atmosphere of nitrogen, at room temperature for 3 hours. After reaction, triethylamine was removed, and the product was extracted with toluene. The resulting product was washed with 1 N-hydrochloric acid and then with water, and was purified with silica gel column, followed by recrystalizing four times with ethanol to obtain 1.87 g of white crystal of Compound No.76 in Table 2, within the scope of the present invention.

IR spectrum, H-NMR spectrum and F-NMR spectrum of this compound were as shown in Fig.2(a), Fig.2(b) and Fig.2(c), respectively.

| Elemental analysis,% | | | | | |
|---|---|---|---|---|---|
| | Observed | C:77.11 | H:8.87 | N:2.93 | F:7.91 |
| | Theoretical | C:77.02 | H:8.90 | N:2.90 | F:7.87 |

Example 3 [Compound No.18]

1) Into 100 ml of methanol, were dissolved 5.00 g of 2,3-difluorophenol; and 5.78 g of sodium iodide and 1.54 g of sodium hydroxide were added and cooled to a temperature below 5°C, followed by adding thereto dropwise 90 ml of 5% aqueous solution of sodium hypochlorite at a temperature of 3-5°C and then continuing stirring for additional 1 hour. Thereafter, 40 ml of 10% aqueous solution of sodium thiosulfate were added thereto, and the product was neutralized with hydrochloric acid and extracted with ether. The ether-containing layer was washed with water, and then recrystalized twice with hexane to obtain 6.40 g of 2,3-difluoro-4-iodophenol.

2) In the same manner as in 2) of Example 1, 2.37 g of 2,3-difluoro-4-iodophenol and 1.51 g of n-hexylbromide were reacted to obtain 2.57 g of 2,3-difluoro-4-iodo-n-hexyloxybenzene.

3) In the same manner as in 2) of Example 1, 20.00 g of p-iodophenol and 19.69 g of n-decylbromide were reacted to obtain 27.81 g of p-iodo-n-decyloxybenzene.

4) In 200 ml of triethylamine, 27.81 g of p-iodo-n-decyloxybenzene and 7.79 g of 3-methyl-1-butyne-3-ol were reacted in the presence of 270 mg of dichlorobistriphenylphosphine palladium$^{II}$ and 68 mg of cuprous iodide, within an atmosphere of nitrogen, at room temperature for 5 hours. After reaction, triethylamine was removed, and the product was extracted with hexane. The hexane-containing layer was washed with 1 N-hydrochloric acid and then with water, followed by removing hexane under reduced pressure to obtain 20.02 g of an oily compound (e) represented by the formula:

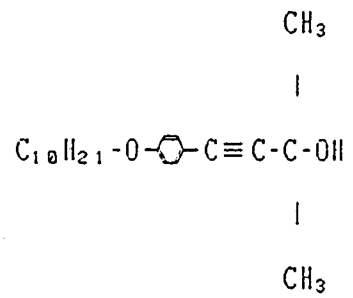

(e)

5) Into 300 ml of unhydrous toluene, were dissolved 20.2 g of the compound (e), followed by adding thereto 2.79 g of sodium hydroxide powder and then heating under reflux for an hour. After reaction, toluene was removed, and the product was extracted with ether and washed with water, followed by distilling off ether under reduced pressure to obtain 13.08 g of an oily compound (f) represented by the formula:

$C_{10}H_{21}$-O-Ph-C≡CH (f)

6) In 50 ml of triethylamine, 2.44 g of the compound (f) and 2.57 g of 2,3-difluoro-4-iodo-n-hexyloxybenzene were reacted in the presence of 26 mg of dichlorobistriphenylphosphine palladium$^{II}$ and 7 mg of cuprous iodide, within an atmosphere of nitrogen, at room temperature for 10 hours. After reaction, triethylamine was removed; and the product was extracted with toluene, and washed with 1 N-hydrochloric acid and then

with water. Thereafter, the product was purified with silica gel column, and then recrystalized four times with ethanol to obtain 1.81 g of white crystal of Compound No.18 in Table 1, within the scope of the present invention.

IR spectrum, H-NMR spectrum and F-NMR spectrum of this compound were as shown in Fig.3(a), Fig.3(b) and Fig.3(c), respectively.

| Elemental analysis,% | Observed | C:76.53 | H:8.55 | F:8.17 |
|---|---|---|---|---|
| | Theoretical | C:76.60 | H:8.51 | F:8.09 |

Example 4 [Compound No.58]

1) In the same manner as in 2) of Example 1, 5.00 g of 2,3-difluoro-4-iodo-n-hexyloxybenzene as prepared in 1) of Example 3 and 2.09 g of ethylbromide were reacted to obtain 4.45 g of 2,3-difluoro-4-iodo-ethoxybenzene.

2) In the same manner as in 5), 6) and 7) of Example 1 except that 1-pentyne was used instead of 1-octyne, were obtained 3.87 g of 2-n-pentyl-5-iodopyridine.

3) In the same manner as in 4) and 5) of Example 3 except that 3.87 g of 2-n-pentyl-5-iodopyridine was used instead of p-iodo-n-decyloxybenzene, were obtained 1.95 g of an oily compound (g) represented by the formula:

$$C_5H_{11}\text{-}x^4\text{-}C{\equiv}CH \qquad (g)$$

4) In 50 ml of triethylamine, 3.20 g of 2,3-difluoro-4-iodo-ethoxybenzene and 1.95 g of the compound (g) were reacted in the presence of 39 mg of dichlorobistriphenylphosphine palladium$^{II}$ and 10 mg of cuprous iodide, within an atmosphere of nitrogen, at room temperature for 8 hours. After reaction, triethylamine was removed; and the product was extracted with toluene, and washed with 1 N-hydrochloric acid and then with water. Thereafter, the product was purified with silica gel column, and then recrystalized four times with methanol to obtain 1.48 g of white crystal of Compound No.58 in Table 2, within the scope of the present invention.

IR spectrum, H-NMR spectrum and F-NMR spectrum of this compound were as shown in Fig.4(a), Fig.4(b) and Fig.4(c), respectively.

| Elemental analysis,% | Observed | C:73.02 | H:6.39 | N:4.24 | F:11.50 |
|---|---|---|---|---|---|
| | Theoretical | C:72.95 | H:6.38 | N:4.26 | F:11.55 |

Examples 5-8 [Compounds Nos.72, 75, 80 and 84]

In the same manner as Example 1, were prepared Compounds No.72, No.75, No.80 and No.84 in Table 2, within the scope of the present invention.

Examples 9-15 [Compounds Nos.7, 11, 12, 16, 19, 20 and 24]

In the same manner as Example 3, were prepared Compounds No.7, No.11, No.12, No.16, No.19, No.20 and No.24 in Table 1, within the scope of the present invention.

Example 16 [Compound No.168]

1) To 200 ml of ethanol containing 20.00 g of 2-bromo-5-nitropyridine, were added 1.00 g of platinum dioxide as catalyst, and reduction were carried out under stirring at room temperature under normal pressure within an atmosphere of hydrogen. After confirming that no absorption of hydrogen was observed, the catalyst was filterred off, followed by distilling off ethanol under reduced pressure to obtain 19.34 g of 2-bromo-5-aminopyridine.

2) Into 500 ml of water containing 77.00 g of 36% hydrochloric acid, were added 15.34 g of 2-bromo-5-aminopyridine, and cooled to a temperature below 5°C. A solution of 5.04 g of sodium nitrite in 35 ml of

water was added thereto dropwise at a temperature below 5°C, followed by continuing stirring for additional 1 hour. Then a solution of 60.00 g of potassium iodide in 70 ml of water was added thereto, and the temperature restored gradually to room temperature, followed by continuing stirring until no generation of nitrogen was observed. Thereafter, the solution was alkalized with sodium hydroxide and extracted with hexane. The hexane-containing layer was washed with water, then with an aqueous sodium hydrogen sulfite and again with water, followed by treating with silica gel column to obtain 18.45 g of oily 2-bromo-5-iodopyridine.

3) In 100 ml of triethylamine, 11.20 g of 2-bromo-5-iodopyridine and 6.36 g of 1-nonyne were reacted in the presence of 137 mg of dichlorobistriphenylphosphine palladium$^{II}$ and 34 mg of cuprous iodide, within an atmosphere of nitrogen, at room temperature for 7 hours. After reaction, the product was extracted with hexane, and washed with water, followed by removing hexane under reduced pressure and then treating the product with silica gel column to obtain 9.98 g of a compound (h) represented by the formula:

$$C_7H_{15}\text{-}C\equiv C\text{-}X^3\text{-}Br \qquad (h)$$

4) In the same manner as in the above 1), instead of 2-bromo-5-nitropyridine, 9.98 g of the compound (h) were hydrogenated to obtain 8.23 g of 2-bromo-5-n-nonylpyridine.

5) In 50 ml of triethylamine, 1.31 g of 2-bromo-5-n-nonylpyridine and 129 g of the compound (d) as prepared in 2) of Example 2 were reacted in the presence of 16 mg of dichloroblstriphenylphosphine palladium$^{II}$, 4 mg of cuprous iodide and 24 mg of triphenylphosphine, within an atmosphere of nitrogen, under heating to reflux for 3 hours. After reaction, triethylamine was removed; and the product was extracted with toluene, and washed with 1 N-hydrochloric acid and then with water. Thereafter, the product was purified with silica gel column, and then recrystalized four times with ethanol to obtain 1.15 g of white crystal of Compound No.168 in Table 3, within the scope of the present invention, the structure of which was confirmed by IR, H-NMR and F-NMR spectra and elemental analysis.

| | | | | | |
|---|---|---|---|---|---|
| IR ($cm^{-1}$) | 2924 | 2854 | 2216 | 1632 | 1516 |
| | 1470 | 1307 | 1087 | 820 | 723 |

| | | | | | |
|---|---|---|---|---|---|
| H-NMR (ppm) | 0.83-0.96(m,6H) 1.17-1.40(m,2H) 1.40-1.53 (m,2H) 1.53-1.68(m,2H) 1.76-1.90(m,2H) 2.63(t,2H) 4.06(t,2H) 6.67-6.77(m,1H) 7.22-7.32(m,1H) 7.44-7.54(m,2H) 8.45(d,1H) | | | | |
| $^{19}$F-NMR (ppm) | -82.63(dd,1F) -57.21(dd,1F) | | | | |
| Elemental analysis,% | Observed | C:77.15 | H:8.97 | N:2.86 | F:7.81 |
| | Theoretical | C:77.02 | H:8.90 | N:2.90 | F:7.87 |

Example 17 [Compound No.172]

In the same manner as Example 16, were prepared Compound No.172 in Table 3, within the scope of the present invention.

Phase transition temperatures of compounds obtained in Examples 1-17 were as shown in Table 12.

In Table 12 and also hereinafter, are used the following abbreviated words:

Cry : crystal phase;
$S_A$ : smectic A phase;
$S_C$ : smectic C phase;
$S_C$* : ciral smectic C phase;
Nem : nematic phase;
Ch : cholesteric phase;

Iso : isotropic liquid phase;
( ) : monotropic phase;
: the phase is present;
- : the phase is not present.

Table 12

| Example No. | Compound No. | Cry | | $S_C$ ($S_C^*$) | | $S_A$ | | Nem (Ch) | | Iso |
|---|---|---|---|---|---|---|---|---|---|---|
| 1 | 70 | • | 23.7 | • | 43.0 | – | | • | 51.2 | • |
| 2 | 76 | • | 38.4 | • | 53.1 | – | | • | 56.1 | • |
| 3 | 18 | • | 32.5 | • | 50.4 | – | | • | 79.7 | • |
| 4 | 58 | • | 55.8 | – | | – | | (• | 39.4) | • |
| 5 | 72 | • | 46.3 | • | 53.0 | • | 55.9 | – | | • |
| 6 | 75 | • | 37.1 | • | 47.8 | – | | • | 55.5 | • |
| 7 | 80 | • | 34.2 | • | 50.0 | – | | • | 54.7 | • |
| 8 | 84 | • | 23.1 | • | 26.7 | – | | • | 31.0 | • |
| 9 | 7 | • | 41.0 | • | 46.5 | – | | • | 79.5 | • |
| 10 | 11 | • | 59.1 | (• | 53.8) | – | | • | 84.1 | • |
| 11 | 12 | • | 51.7 | • | 57.1 | – | | • | 84.1 | • |
| 12 | 16 | • | 56.0 | • | 62.5 | – | | • | 81.8 | • |
| 13 | 19 | • | 41.9 | • | 54.6 | – | | • | 78.8 | • |
| 14 | 20 | • | 37.2 | • | 65.2 | – | | • | 84.6 | • |
| 15 | 24 | • | 19.8 | • | 54.5 | – | | • | 65.8 | • |
| 16 | 168 | • | 56.6 | (• | 55.3) | – | | • | 63.0 | • |
| 17 | 172 | • | 51.4 | • | 57.5 | – | | • | 62.3 | • |

Example 18

A composition comprising 80 % of Compound No.76 and 20 % of a compound represented by the formula (i) and having phase transition temperatures of 47.8°C[Cry-$S_C$], 47.9°C[$S_C$-$S_A$] and 51.9°C[$S_A$-Iso] was prepared, and phase transition temperatures thereof were determined.

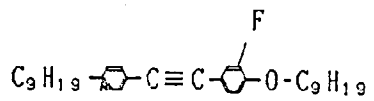

(i)

This composition showed a series of Iso-Nem-$S_A$-$S_C$ phases, having phase transition temperatures of 40.0°C [Cry-$S_C$], 52.5°C[$S_C$-$S_A$], 53.5°C[$S_A$-Nem] and 54.9°C[Nem-Iso]; and reduction of the minimum temperature of $S_C$ phase and elevation of the maximum temperature of $S_C$ phase were observed, as compared with each component.

Example 19

A composition comprising 20 % of Compound No.76 and 80 % of 2-p-n-nonyloxyphenyl-5-n-nonylpyrimidine having phase transition temperatures of 35.6°C[Cry-$S_C$], 52.5°C[$S_C$-$S_A$] and 53.5°C[$S_A$-Nem] was prepared, and phase transition temperatures thereof were determined.

This composition showed a series of Iso-Nem-$S_A$-$S_C$ phases, having phase transition temperatures of 22.2°C [Cry-$S_C$], 64.0°C[$S_C$-$S_A$], 67.3°C[$S_A$-Nem] and 68.7°C[Nem-Iso]; and reduction of the minimum temperature of $S_C$ phase and elevation of the maximum temperature of $S_C$ phase were observed, as compared with each component.

Example 20

A composition comprising 10 % of Compound No.168 and 90 % of 2-p-n-nonyloxyphenyl-5-n-nonylpyrimidine was prepared, and phase transition temperatures thereof were determined.

This composition showed a series of Iso-Nem-$S_A$-$S_C$ phases, having phase transition temperatures of 27.0°C [Cry-$S_C$], 68.0°C[$S_C$-$S_A$], 71.2°C[$S_A$-Nem] and 72.6°C[Nem-Iso]; and reduction of the minimum temperature of $S_C$ phase and elevation of the maximum temperature of $S_C$ phase were observed, as compared with each component.

Example 21

A composition comprising 11 % of Compound No.172 and 89 % of 2-p-n-octyloxyphenyl-5-n-decylpyrimidine having phase transition temperatures of 40.6°C[Cry-$S_C$], 69.6°C[$S_C$-$S_A$] and 74.6°C[$S_A$-Iso] was prepared, and phase transition temperatures thereof were determined.

This composition showed a series of Iso-Nem-$S_A$-$S_C$ phases, having phase transition temperatures of 38.0°C [Cry-$S_C$], 70.8°C[$S_C$-$S_A$], 71.6°C[$S_A$-Nem] and 72.2°C[Nem-Iso]; and reduction of the minimum temperature of $S_C$ phase and elevation of the maximum temperature of $S_C$ phase were observed, as compared with each component.

Example 22 [Compound No.306]

1) Into 200 ml of DMSO, were dissolved 25.00 g of p-iodophenol, and a solution of 8.40 g of sodium hydroxide in 30 ml of water and 17.80 g of n-hexylbromide were added and stirred for 4 days at room temperature. The resulting reaction product was extracted with hexane and then washed with water, followed by distilling off hexane to obtained 31.00 g of oily p-n-hexyloxy-iodobenzene.

2) 120 ml of ether solution of grignard reagent,that prepared from 15.00 g of p-n-hexyloxy-iodobenzene and magnesium, and 120 ml of dry THF solution of 14.00 g of 2,5-dibromopyridine were reacted in the presence of 357 mg of dichlorobistriphenylphosphinobutane palladium, within an atmosphere of nitrogen, at room temperature for 5 hours. Reaction was stopped with water, then the product was extracted with ether. The resulting product was washed with 1 N-hydrochloric acid and then with water, followed by distilling off ether. Thereafter, the product was recrystalized with methanol to obtain 8.90 g of the compound (j) represented by the formula;

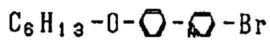

(j)

3) In 50 ml of triethylamine, 1.10 g of the compound (j) and 1.16 g of the compound (k) represented by the

formula;

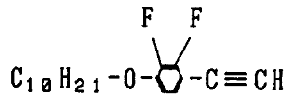

(k)

which was obtained in the same manner as in 1),2),3) and 4) of Example 1 exept that n-decylbromide was used instead of n-octylbromide, were reacted in the presence of 16 mg of dichlorobistriphenylphosphine palladiun II, 4 mg of cuprous iodide and 24 mg of triphenylphosphine, within an atmosphere of nitrogen, under reflux for 7 hours. After reaction, trietylamine was removed, and product was extracted with toluene. The resulting product was washed with 1 N-hydrochloric acid and then with water, and was purified with silica gel column, followed by recrystalizing four times with ethanol to obtain 0.40 g of white crystal of Compound No.306 in Table 6, within the scope of the present invention, the structure of which was confirmed by H-NMR and F-NMR spectra and elemental analysis.

| | | | | | |
|---|---|---|---|---|---|
| H-NMR (ppm) | 0.81-0.97(m,6H) 1.18-1.40(m,16H) 1.40-1.54(m,4H) 1.74-1.88(m,4H) 3.96-4.09(m,4H) 6.65-6.75(m,1H) 6.99(d,2H) 7.15-7.25(m,1H) 7.65(d,1H) 7.82(dd,1H) 7.98(d,2H) 6.78(d,1H) | | | | |
| F-NMR (ppm) | -82.44(dd,1F) -57.54(dd,1F) | | | | |
| Elemental analysis,% | Observed | C:76.82 | H:7.84 | N:2.55 | F:6.98 |
| | Theoretical | C:76.78 | H:7.86 | N:2.56 | F:6.95 |

Example 23 [Compound No.333]

1) In 40 ml of ethanol, 0.40 g of the Compound No.306 in Table 6 which was obtained Example 22, were added 100 mg of 5 % palladium-carbon as catalyst, was hydrogenated at room temperature under the normal pressure within an atmosphere of hydrogen. After reaction, ethanol was removed, and the product was recrystalized with hexane to obtain 0.20 g of white crystal of Compound No. 333 in Table 7, within the scope of the present invention, the structure of which was confirmed by IR, H-NMR and F-NMR spectra end elemental analysis.

| | | | | | |
|---|---|---|---|---|---|
| IR ($cm^{-1}$) | 2924 2856 1607 1518 1475 1396 1247 1176 1083 824 | | | | |
| H-NMR (ppm) | 0.80-0.98(m,6H) 1.20-1.40(m,16H) 1.40-1.53(m,4H) 1.71-1.87(m,4H) 2.82-2.94(m,4H) 6.56-6.77(m,2H) 6.97(d,2H) 7.45(dd,1H) 7.55(d,1H) 7.91(d,2H) 8.41(d,1H) | | | | |
| F-NMR (ppm) | -83.64(dd,1F) -66.70(dd,1F) | | | | |
| Elemental analysis,% | Observed | C:76.34 | H:8.51 | N:2.56 | F:6.89 |
| | Theoretical | C:76.23 | H:8.53 | N:2.54 | F:6.90 |

Example 24 [Compound No.381]

1) Into 80 ml of pyridine, were dissolved 2.81 g of the compound (l) represented by the formula;

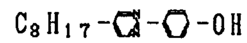

(l)

was cooled to a temperature below 5°C, followed by adding thereto dropwise 2.27 ml of trifluoromethanesulfonic anhydride at a temperature below 5°C, and then continuing stirring for additional 2 days at room temperature. After reaction, the product was extracted with toluene. The resulting product was washed with 1 N-hydrochloric acid and then with water, and was treated with silica gel column, followed by distilling off toluene to obtained 3.50 g of the compound (m) represented by the formula;

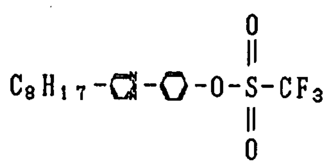

(m)

2) In 50 ml of trietylamine, 1.50 g of the compound (m) and 1.90 g of the compound (n) represented by the formula;

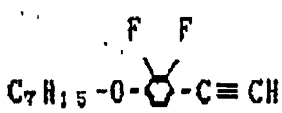

(n)

which was prepared in the same manner as in 1),2), 3) and 4) of Example 1 exept that n-heptylbromide was used instead of n-octylbromide, were reacted in the presence of 38 mg of dichlorobistriphenylphosphine palladium II, 9 mg of cuprous iodide and 76 mg of triphenylphosphine, within an atmosphere of nitrogen, under reflux for 8 hours. After reaction, trietylamine was removed, and product was extracted with toluene. The resulting product was washed with 1 N-hydrochloric acid and then with water, and was purified with silica gel column, followed by recrystalizing four times with ethanol to obtain 1.30 g of white crystal of Compound No.381 in Table 8, within the scope of the present invention, the structure of which was confirmed by IR, H-NMR and F-NMR spectra and elemental analysis.

| | | | | | |
|---|---|---|---|---|---|
| IR ($cm^{-1}$) | 2928 2858 1632 1586 1504 1483 1431 1301 1212 1087 859 797 | | | | |
| H-NMR (ppm) | 0.82-0.94(m,6H) 1.18-1.41(m,14H) 1.41-1.52(m,4H) 1.58-1.70(m,2H) 1.76-1.88(m,2H) 2.62(t,2H) 4.05 (t,2H) 6.66-6.75(m,1H) 7.16-7.24(m,1H) 7.64(d,2H) 8.41(d,2H) 8.62(s,2H) | | | | |
| F-NMR (ppm) | -82.59(dd,1F) -57.64(dd,1F) | | | | |
| Elemental analysis,% | Observed | C:76.51 | H:8.01 | N:5.37 | F:7.35 |
| | Theoretical | C:76.15 | H:8.08 | N:5.38 | F:7.31 |

Example 25 [Compound No.100]

1) In 50 ml of triethylamine, 1.00 g of 1-decyne and 2.70 g of 2,5-dibromopyridine were reacted in the presence of 23 mg of dichlorobistriphenylphosphine palladium II, 6 mg of cuprous iodide and 35 mg of triphenylphosphine, within an atmosphere of nitrogen, under reflux for 5 hours. After reaction, trietylamine was removed, and product was extracted with toluene. The resulting product was washed with 1 N-hydrochloric acid and then with water, followed by distilling off toluene under reduced pressure to obtain 1.60 g of an oily compound (o) represented by the formula;

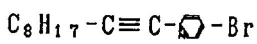

(o)

2) In 50 ml of triethylamine, 1.50 g of compound (o) and 1.63 g of compound (b) as prepared in 1),2), 3) and 4) of Example 1 were reacted in the presence of 16 mg of dichlorobistriphenylphosphine palladium II, 4 mg of cuprous iodide and 24 mg of triphenylphosphine, within an atmosphere of nitrogen, under reflux for 8 hours. After reaction, trietylamine was removed, and product was extracted with toluene. The resulting product was washed with 1 N-hydrochloric acid and then with water, and was purified with silica gel column, followed by recrystalizing twice with methanol and once with hexane to obtain 1.11 g of white crystal of Compound No.100 in Table 2, within the scope of the present invention, the structure of which was confirmed by IR, H-NMR and F-NMR spectra and elemental analysis.

| | | | | | |
|---|---|---|---|---|---|
| IR ($cm^{-1}$) | 2922 2856 2220 1632 1514 1491 1303 1094 847 814 | | | | |
| H-NMR (ppm) | 0.81-0.95(m,6H) 1.18-1.39(m,16H) 1.39-1.51(m,4H) 1.55-1.69(m,2H) 1.75-1.88(m,2H) 2.42(t,2H) 4.05(t,2H) 6.65-6.75(m,1H) 7.11-7.21(m,1H) 7.33(d,1H) 7.71(dd,1H) 8.66(d,1H) | | | | |
| F-NMR (ppm) | -82.58(dd,1F) -57.64(dd,1F) | | | | |
| Elemental analysis,% | Observed | C:77.58 | H:8.16 | N:2.95 | F:7.89 |
| | Theoretical | C:77.66 | H:8.14 | N:2.92 | F:7.93 |

Example 26 [Compound No.433]

1) To 10.00 g of 2,3-difluorophenol dissolved in 100 ml of DMSO, were added 4.00 g of sodium hydroxide and 15.95 g of n-decylbromide, and stirred for 5 days at room temperature. The resulting reaction product was extracted with hexane and then washed with water, followed by distilling off hexane to obtain 17.63 g of oily 2,3-difluoro-n-decyloxybenzene.

2) To 17.00 g of 2,3-difluoro-n-decyloxybenzene dissolved in 61.5 ml of solvent (acetic acid;50ml, water;10ml and sulfuric acid;1.5ml), were added 6.40 g of iodine and 2.90 g of periodic acid, and stirred for 5 hours at 80 °C. The resulting reaction product was extracted with hexane and then washed with water, followed by distilling off hexane to obtain 23.68 g of oily 2,3-difluoro-4-iodo-n-decyloxybenzene.

3) In 100 ml of triethyl amine, 23.68 g of 2,3- difluoro-4-iodo-n-decyloxybenzene and 7.50 g of 3-methyl-1-butyne-3-ol were reacted in the presence of 126 mg of dichlorobistriphenyl phosphine palladium(II) and 32 mg of cuprous iodide, within an atmosphere of nitrogen at room temperature for 6 hours. Then triethylanine was removed, and the product was extracted with hexane. The organic layer was washed with 1N hydrochloric acid and then with water, followed by distilling off hexane under reduced pressure to obtain 18.26g of an oily compound (p) represented by the formula:

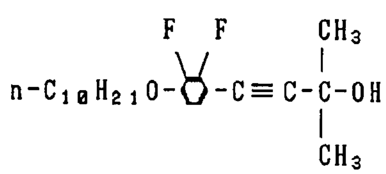

(p)

4) Into 500 ml of anhydrous toluene, were dissolved 18.26g of the compound (p), followed by adding thereto 4.00 g of sodium hydroxide powder and then heating under reflux for an hour. After reaction, toluene was removed, and the product was extracted with ether and washed with water, followed by distilling off ether under reduced pressure to obtain 10.55 g of an oily 2,3-difluoro-4-n-decyloxyphenylacetylene.

5) To 5.00 g p-bromobenzoic acid was added 10 ml of thionyl chloride and refluxed for 6 hours. Then unreacted thionyl chloride was removed and 30 ml of anhydrous toluene was added. To 2.8 g of (S)-(-)-4-methylhexanol and 10 ml of pyridine dissolved in 20 ml of anhydrous toluene, above toluene solution contained acid chloride was added dropwise under cooling with ice bath, and stirred for 5 hours at 80°C, followed by washing with 1N hydrochloric acid and then with water. Toluene was removed by evaporation to obtain 8.83 g of an oily compound (q) represented by formula:

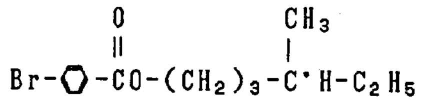 (q)

6) In 80 ml of triethylamine, 2.94 g of 2,3-difluoro-4-n-decyl oxyphenylacetylene and 2.98 g of the compound (q) were reacted in the presence of 36 mg of dichlorobistriphenylphosphine palladium (II), 9 mg of cuprous iodide and 72 mg of triphenylphosphine, within an atmosphere of nitrogen, under reflux for 8 hours. After reaction, triethylamine was removed, and the product was extracted with toluene. The organic layer was washed with 1N-hydrochloric acid and then with water, and was purified with silica gel column, followed by recrystalization three times with methanol to obtain 2.23 g of white crystal of Compound No.433 in Table 11, within the scope of the present invention.

| | | | | |
|---|---|---|---|---|
| IR ($cm^{-1}$) | 2922 2210 1717 1605 1506 1470 1272 1091 799 768 | | | |
| H-NMR (ppm) | 0.82-0.94(m,9H) 1.08-1.50(m,19H) 1.69-1.89 (m,4H) 4.05(t,2H) 4.30(t,2H) 6.67-6.75 (m,1H) 7.16-7.24(m,1H) 7.59(d,2H) 8.02(d,2H) | | | |
| $^{19}F$-NMR (ppm) | -82.66(dd,1F) -57.65(dd,1F) | | | |
| Elemental analysis,% | Observed | C:75.17 | H:8.07 | F:7.51 |
| | Theoretical | C:75.00 | H:8.20 | F:7.42 |

Example 27 [Compound No.429]

1) To 20.00 g of decanoic acid chloride and 14.70 g of anhydrous aluminium chloride dissolved in 50 ml of chloroform, 22.50 g of iodobenzene dissolved in 20 ml of chloroform were added dropwise under,cooling with ice bath, and stirred for 6 hours at room temperature, followed by washing with water and then distilling off chloroform. Thereafter, the product was recrystalized with methanol to obtain 14.95 g of white crystal of compound (r) represented by the formula:

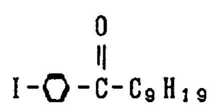 (r)

2) In the same manner as in 1),2),3) and 4) of Example 25 except that n-nonylbromide was used instead of n-decylbromide, were obtained 9.62 g of an oily 2,3-difluoro-4-n-nonyloxyphenylacetylene.

3) In 80 ml of triethylamine, 2.80 g of 2,3-difluoro-4-n-nonyl oxyphenylacetylene and 3.58 g of the compound (r) were reacted in the presence of 20 mg of dichlorobistriphenylphosphine palladium (II) and 5 mg of cuprous iodide, within an atmosphere of nitrogen at room temperature for 6 hours. After reaction, triethylamine was removed, and the product was extracted with toluene. The organic layer was washed with 1N-hydrochloric acid and then with water, and was purified with silica gel column, followed by recrystalization three times with methanol to obtain 2.76 g of white crystal of Compound No.429 in Table 11, within the scope of the present invention.

| | | | | |
|---|---|---|---|---|
| IR ($cm^{-1}$) | 2920 2854 2210 1686 1601 1518 1470 1301 1083 | | | |
| H-NMR (ppm) | 0.87-0.95(m,6H) 1.16-1.51(m,24H) 1.65-1.88 (m,4H) 2.95(t,2H) 4.06(t,2H) 6.67-6.75 (m,1H) 7.15-7.23(m,1H) 7.59(d,2H) 7.94(d,2H) | | | |
| $^{19}F$-NMR (ppm) | -82.64(dd,1F) -57.63(dd,1F) | | | |
| Elemental analysis,% | Observed | C:77.53 | H:8.39 | F:7.61 |
| | Theoretical | C:77.65 | H:8.63 | F:7.45 |

Example 28 [Compound No.431]

1) To 1.34 g of magnesium, 5.00g of (S)-(-)-4-methyl-1-bromohexane and 5.25 g of 1,2-dibromoethane dissolved in 30 ml of anhyrdrous ether were added dropwise at the rate the ether was mildly refluxing, followed by refluxing for 2 hours. Then 120 ml of anhydrous benzene was added, and about 30 ml of solvent was distilled off, followed by adding 4.07 g of 4-cyanobromobenzene and then refluxing for 5 hours. Thereafter, the reaction mixture was cooled below 10°C with ice bath. And then 20 ml of 6 N-hydrochloric acid was added, followed by refluxing for 6 hours. After reaction, the organic layer was washed with water, followed by distilling off solvent. Thereafter, the product is recrystalized with methanol to obtain 3.79 g of a compound (s) represented by the formula:

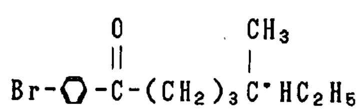

(s)

2) In 80 ml of triethylamine, 2.94 g of 2,3-difluoro-4-n-decyl oxyphenylacetylene and 2.83 g of the compound (s) were reacted in the presence of 36 mg of dichlorobistriphenylphosphine palladium (II), 9 mg of cuprous iodide and 72 mg of triphenylphosphine wit hin an atmosphere of nitrogen, under reflux for 8 hours. Afterrea ction, triethylamine was removed, and the product was extracted with toluene. The organic layer was washed with 1N-hydrochloric acid and then with water, and was purified with silica gel column followed by recrystalization three times with methanol to obtain 2.13 g of white crystal of Compound No.431 in Table 11, within the scope of the present invention.

| | | | | |
|---|---|---|---|---|
| IR ($cm^{-1}$) | 2926 2210 1680 1603 1520 1481 1408 1305 1083 799 | | | |
| H-NMR (ppm) | 0.81-0.92(m,9H) 1.05-1.50(m,19H) 1.60-1.88 (m,4H) 2.93(t,2H) 4.06(t,2H) 6.68-6.75 (m,1H) 7.15-7.22(m,1H) 7.59(d,2H) 7.94(d,2H) | | | |
| $^{19}F$-NMR (ppm) | -82.64(dd,1F) -57.64(dd,1F) | | | |
| Elemental analysis,% | Observed | C:77.53 | H:8.49 | F:7.43 |
| | Theoretical | C:77.42 | H:8.47 | F:7.66 |

Example 29 [Compound No.430]

1) To 15.00 g of 2,3-difluorophenol dissolved in 150 ml of DMSO, were added 6.92 g of sodium hydroxide and 14.00 g of benzylchloride, and stirred for 3 days at room temperature. The resulting reaction product was extracted with hexane and then washed with water, followed by distilling off hexane to obtain 24.11 g of 2,3-difluorobenzyloxybenzene.

2) To 24.11 g of 2,3-difluorobenzyloxybenzene and 25.43 g of N,N,N',N'-tetramethylethylenediamine dissolved in 200ml of anhydrous tetrahydrofuran, 74 ml of n-buthyl lithium solution in hexane, contained 15% by weight, was added dropwise under cooling with dry ice acetone bath, and stirred for 3 hours below -55°C, followed by bubbling carbon dioxide for an hour below -55°C. After reaction, tetrahydrofuran was removed, then water was added and acidified by hydrochloric acid. The resulting precipitate was recrystalized with toluene to obtain 15.04 g of 2,3-difluoro-4-benzyloxybenzoic acid.

3) To 15.04 g of 2,3-difluoro-4-benzyloxybenzoic acid was added 20 ml of thionyl chloride and refluxed for 6 hours. Then unreacted thionyl chloride was removed and 150 ml of anhydrous toluene was added. To this solution, ammonia gas was bubbled under cooling with ice bath. The resulting white precipitate was filterred off, followed by washing with water and drying to obtain 14.35 g of 2,3-difluoro-4-benzyloxybenzamide.

4) To 14.35 g of 2,3-difluoro-4-benzyloxybenzamide was added 50 ml of thionyl chloride and refluxed for 12 hours. Then unreacted thionyl chloride was removed. The resulting precipitate was extracted with toluene and washed with water. The toluene was removed by evaporation, followed by recrystalizing with hexane to obtain 8.02 g of 2,3-difluoro-4-benzyloxycyanobenzene.

5) To 0.83 g of magnesium, 3.55g of n-nonylbromide and 3.22 g of 1,2-dibromoethane dissolved in 20 ml of anhyrdrous ether were added dropwise at the rate the ether was mildly refluxing, followed by refluxing for 2 hours. Then 80 ml of anhydrous benzene was added, and about 20 ml of solvent was distilled off, followed by adding 2.10 g of 2,3-difluoro-4-benzyloxycyanobenzene and then refluxing for 5 hours. Thereafter, the reaction mixture was cooled below 10°C with ice bath. And then 10 ml of 6N-hydrochloric acid was added, followed by refluxing for 6 hours. After reaction, the organic layer was washed with water, followed by distillating off solvent. Thereafter, the product is recrystalized with methanol to obtain 2.07 g of a compound (t) represented by the formula:

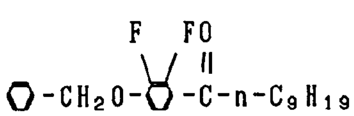

(t)

6) To 30 ml of ethanol containing 2.07 g of the compound (t), were added 0.5 g of 5% palladium-carbon as catalyst, and hydrogenation was carried out at room temperature under normal pressure within an

atmosphere of hydrogen. After confirming that no absorption of hydrogen was observed, the catalyst was filterred off, followed by distillating off ethanol under reduced pressure. Thereafter, the product is recrystalized with hexane to obtain 1.26 g of a compound (u) represented by the formula:

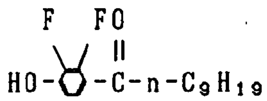

(u)

7) To 1.26 g of the compound (u) dissolved in 30 ml of anhydrous pyridine, 1.38 g of trifluoromethanesulfonic anhydride was added dropwise under cooling with ice bath, and stirred for a day at room temperature. After reaction, the product was extracted with hexane and washed with 1N-hydrochloric acid and then with water. Thereafter, the hexane was removed by evaporation to obtain 1.48 g of a compound (v) represented by the formula:

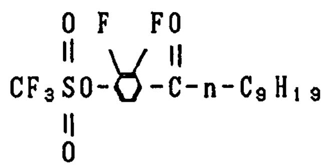

(v)

8) In 30 ml of triethylamine, 0.88 g of 4-n-nonyloxyphenyl acetylene and 1.48 g of the compound (v) were reacted in the presence of 12 mg of dichlorobistriphenylphosphine palladium (II) , 4 mg of cuprous iodide and 24 mg of triphenylphosphine within anatmosphere of nitrogen, under reflux for 8 hours. After reaction-,triethylamine was removed, and the product was extracted with toluene. The organic layer was washed with 1N-hydrochloric acid and then with water, and was purified with silica gel column followed by recrystalization three times with methanol to obtain 0.76 g of white crystal of Compound No.430 in Table 11, within the scope of the present invention.

| | | | | |
|---|---|---|---|---|
| IR ($cm^{-1}$) | 2920 2852 2210 1684 1603 1518 1460 1253 820 | | | |
| H-NMR (ppm) | 0.82-0.95(m,6H) 1.17-1.50(m,2H) 1.65-1.85 (m,4H) 2.90-3.00(m,2H) 3.97(t,2H) 6.88(d,2H) 7.25-7.32(m,1H) 7.49(d,2H) 7.52-7.60(m,1H) | | | |
| $^{19}$F-NMR (ppm) | -59.94(d,1F) -58.92(dd,1F) | | | |
| Elemental analysis,% | Observed | C:77.70 | H:8.57 | F:7.41 |
| | Theoretical | C:77.65 | H:8.63 | F:7.45 |

Example 30 [Compound No.411]

1) To 250 ml DMSO containing dissolved therein 15.3 g of 2,3-difluoro-4-bromophenol prepared in the same manner as 1) of Example 3, were added a solution of 2.5 g of sodium hydroxide in 10 ml of water and mixed homogeneously under stirring. Then, 7.9 g of benzyl chloride was added and stirred for 3 days at room temperature. Thereafter, water was added, and the resulting reaction product was extracted with hexane and then washed with water, followed by distilling off hexane. The resulting solid material was recrystalized

with methanol to obtain 16.9 g of 2,3-difluoro-4-benzyloxybenzene.

2) Into a beaker containing finely devided dry ice, were charged quickly a diethyl ether solution of Grignard's reagent prepared from 6.9 g of 1,2-dibromoethane and 12.7 g of 2,3-difluoro-4-benzyloxybenzene. After allowing to stand until the dry ice was evaporated, 1N hydrochloric acid was added to make acidic, and the organic phase was washed with water, followed by removing ether. The resulting solid material was recrystalized with toluene to obtain 4.2 g of white solid 4-benzyloxy-2,3-difluorobenzoic acid.

3) Into 5 ml of thionyl chloride, were dissolved 1.3 g of 4-benzyloxy-2,3-difluorobenzoic acid, and reacted under reflux for 3 hours. After reaction, excess of thionyl chloride was removed with an aspirator to obtain liquid 4-benzyloxy-2,3-difluorobenzoic chloride, which was used without purification in the following reaction in the form of toluene solution.

4) To a mixed solvent of 10 ml anhydrous toluene and 10 ml of anhydrous pyridine, were added 0.7 g of optically active 6-methyl-1-octanol, and cooled to a temperature below 10°C with ice bath. To this solution, was added dropwise a toluene solution of 4-benzyloxy-2,3-difluorobenzoic chloride at a temperature below 10°C, followed by reacting for an hour and then continuing stirring at 80°C for additional 5 hours. Thereafter, the reaction product was cooled to room temperature and was thrown into iced water. The toluene phase was washed with 1N hydrochloric acid, with water, with an aqueous sodium hydrogen carbonate and then with water,followed by removing toluene to obtain 1.8 g of oily optically active 6-methyloctyl 4-benzyloxy-2,3-difluorobenzoate.

5) To 1.4 g of optically active 6-methyloctyl 4-benzyloxy-2,3-difluorobenzoate, were added 30 ml of ethanol and 100 mg of 5% palladium carbon, and hydrogenolysis was carried out. After confirming that no absorption of hydrogen was observed, the catalyst was filtered off, and ethanol was removed. The resulting product was extracted with hexane, followed by washing the hexane-soluble matters with water and then removing hexane to obtain 1.4 g of solid optically active 6-methyloctyl 4-hydroxy-2,3-difluorobenzoate.

6) Into 10 ml of pyridine, were dissolved 1.4 g of optically active 6-methyloctyl 4-hydroxy-2,3-difluorobenzoate, and 1.6 g of trifluoromethanesulfonic anhydride were added dropwise at a temperature below 10°C, followed by continuing stirring at room temperature for 1 day. Thereafter, the reaction product was thrown into iced water, and extracted with toluene. The toluene phase was washed with 1N hydrochloric acid and then with water, followed by removing toluene to obtain 1.7 g of optically active 6-methyloctyloxycarbonylphenyl trifluoromethanesulfonate.

7) In 30 ml of triethylamine, 1.7 g of optically active 6-methyloctyloxycarbonylphenyl trifluoromethanesulfonate and 1.3 g of p-decyloxyphenylacetylene were reacted in the presence of 5 mg of cuprous iodide, 20 mg of dichlorobistriphenylphosphine palladium$^{II}$ and 40 mg of triphenylphosphine, within an atmosphere of nitrogen, under reflux for 6 hours. After reaction, triethylamine was removed, and the product was extracted with toluene. The toluene-containing phase was washed with 1 N-hydrochloric acid and then with water, followed by distilling off toluene and then recrystalyzing thrice the resulting solid with ethanol to obtain 1.2 g of Compound No.411 in Table 10, within the scope of the present invention, the structure of which was confirmed by IR, H-NMR and F-NMR spectra and elemental analysis.

| | |
|---|---|
| IR ($cm^{-1}$) | 2924 2850 2218 1707 1605 1462<br>1311 1253 1172 1023 830 777 |

| H-NMR (ppm) | 0.82-0.93(m,9H) 1.05-1.52(m,23H) 1.73-1.86(m,4H) 3.97(t,2H) 4.35(t,2H) 6.89(d,2H) 7.23-7.30(m,1H) 7.51(d,2H) 7.63-7.70(m,1H) | | | |
|---|---|---|---|---|
| $^{19}$F-NMR (ppm) | -58.2(dd,1F) -58.8(dd,1F) | | | |
| Elemental analysis,% | Observed | C:75.32 | H:8.63 | F:7.23 |
| | Theoretical | C:75.55 | H:8.52 | F:7.04 |

Phase transition temperatures of compounds obtained in Examples 22-30 were as shown in Table 13.

Table 13

| Example No. | Compound No. | Phase transition temperatures (°C) | | | | | | | | |
|---|---|---|---|---|---|---|---|---|---|---|
| | | Cry | | $S_C$ ($S_C^*$) | | $S_A$ | | Nem (Ch) | | Iso |
| 22 | 306 | • | 72.7 | • | 167.0 | • | | • | 193.8 | • |
| 23 | 333 | • | 63.6 | • | 131.3 | – | | – | | • |
| 24 | 381 | • | 99.2 | • | 117.5 | – | | • | 166.4 | • |
| 25 | 100 | • | 74.7 | – | | – | | (• | 62.7) | • |
| 26 | 433 | • | 45.4 | – | | (• | 39.8) | (• | 39.9) | • |
| 27 | 429 | • | 86.0 | (• | 84.8) | • | 104.8 | – | | • |
| 28 | 431 | • | 61.4 | (• | 57.1) | • | 85.3 | – | | • |
| 29 | 430 | • | 77.7 | – | | • | 105.8 | – | | • |
| 30 | 411 | • | 48.8 | (• | 32.1) | • | 53.0 | – | | • |

The present invention provide liquid crystal compounds showing smectic C phase or ciral smectic C phase, which have unexpected effects as follows:

(1) Smectic C phase liquid crystal compounds and ciral smectic C phase liquid crystal compounds according to the invention, having electron-attractive halogen atoms in side or minor axis of the molecule, provide negative dielectric anisotropy, desired in liquid crystal compositions. Liquid crystal compositions comprising liquid crystal compounds according to the invention, showing negative dielectric anisotropy, are capable of improving memory effects in AC stabilize method for driving liquid crystal display elements.

(2) These compounds, showing no other smectic phase in lower temperature side than smectic C phase,

are effective for broadening smectic C temperature range.

(3) These compounds are of low viscosity and capable of reducing viscosity of compositions and improving response time.

(4) These compounds are rigid and provide high alignment.

(5) These compounds have good stability to light, heat and moisture.

(6) Some of these compounds, showing no smectic A phase between smectic C phase and nematic phase, provide larger tilt angle and are also useful as a component adjusting tilt angle.

(7) By blending a liquid crystal compound of this invention showing a serises of Iso-Nem-$S_C$ phases with a liquid crystal compound showing a serises of Iso-$S_A$-$S_C$ phases, there can be obtained liquid crystal compositions of high alignment showing Iso-Nem-$S_A$-$S_C$ phases.

(8) By blending a liquid crystal compound of this invention showing a serises of Iso-Nem-$S_C$ phases with a liquid crystal compound showing a serises of Iso-$S_A$-$S_C$ phases, there can be attained liquid crystal compositions showing the maximum temperature of $S_C$ phase higher than that of each component and therefore effects broadening smectic C temperature range.

Thus, liquid crystal compounds of the present invention are advantageously used in in the production of ferroelectric smectic liquid crystal compositions.

Having described the present invention, it will now be apparent to one skilled in the art that many changes and modifications can be made to the embodiments disclosed while remaining within the spirit and scope of the present invention.

## Claims

1. A smectic C or chiral smectic C liquid crystal compound, represented by the following general formula (1) or (2):

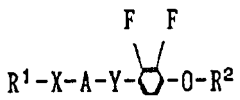

(1)

$R^1$-O-$A^1$-C≡C-$A^2$-CO-$(O)_p$-$R^2$ (2)

wherein $R^1$ and $R^2$ are the same or different alkyl groups containing 1 to 18 preferably 1 to 14 carbon atoms, which may be optically active and may be each unsubstituted or substituted with at least one of F, Cl, alkoxy, trifluoromethyl and cyano groups;
A is a cyclic group selected from the group consisting of Ph, $X^1$, $X^2$, $X^3$; $X^4$, $X^5$, Ph-$X^4$ and $X^1$-Ph, wherein Ph represents 1,4-phenylene group, which may be unsubstituted or substituted with at least one substituent selected from the group consisting of F, Cl, CN and $NO_2$; $X^1$, $X^2$, $X^3$, $X^4$ and $X^5$ represent

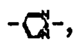, 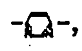,

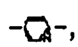, 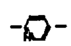 and 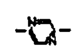 ,

respectively;
X is direct bond, O or -C≡C-, X being O when A is Ph;
Z is -C≡C- or $-CH_2CH_2-$;
one of $A^1$ and $A^2$ is 1,4-phenylene group and the other is 2,3-difluoro-1,4-phenylene group; and p is 0 or 1.

2. A liquid crystal composition, containing as components thereof a mixture of a plurality of crystal compounds, said liquid crystal compounds comprising at least one compound according to claim 1.

3. A composition according to claim 2, which comprises a compound showing chiralsmectic C phase.

4. A composition according to claim 3, which the compound showing chiralsmectic C phase is at least one of optically active 2-(2-methylbutyloxycarbonyl)-naphthalene-6-4′-n-alkoxyphenyl carboxylate, optically

active 2-(1-methylheptyloxycarbonyl)-naphthalene-6-4′-n-alkoxyphenylcarboxylate, optically active 2-(2-methylbutyloxy)-naphthalene-6-4′-n-alkoxyphenylcarboxylate and optically active 2-(1-methylheptyloxy)-naphthalene-6-4′-n-alkoxyphenylcarboxylate.

**5.** A composition according to claim 2 , which comprises a compound showing smectic C phase.

**6.** A composition according to claim 5, in which the compound showing smectic C phase is at least one of 2-4′-alkoxyphenyl-5-alkylpyrimidine and 1-2′-alkyl-5′-pyridyl-2-4′-alkoxy-3′-fluorophenylacetylene.

**7.** A composition according to any one of claims 2 to 4. which further comprises a two-tone dyestuff.

**8.** A liquid crystal display element, containing the liquid crystal composition according to any one of Claims 2 to 7.

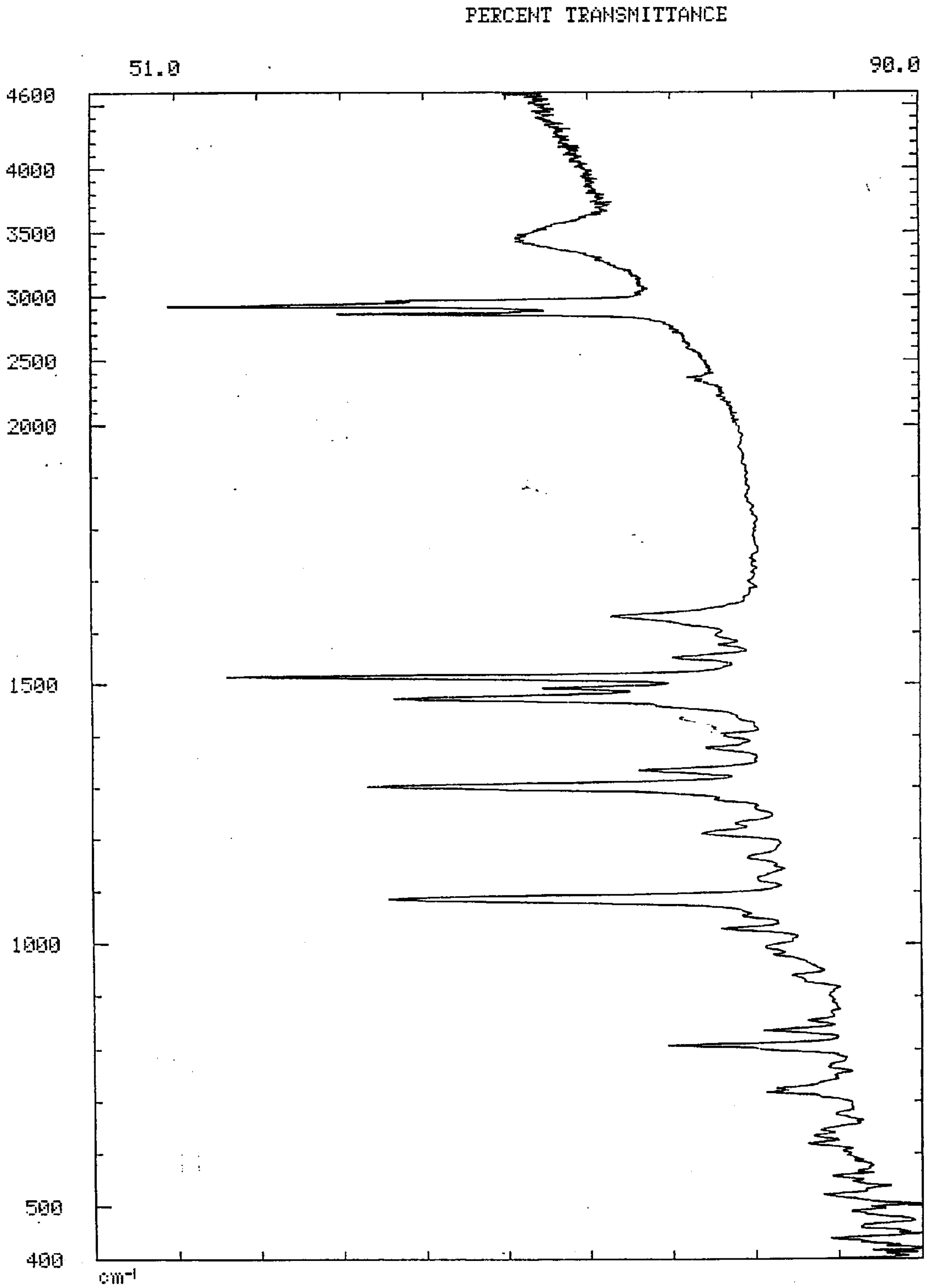
PERCENT TRANSMITTANCE
51.0
90.0
4600
4000
3500
3000
2500
2000
1500
1000
500
400
cm-1

Fig. 1 (a)

Fig.1 (b)

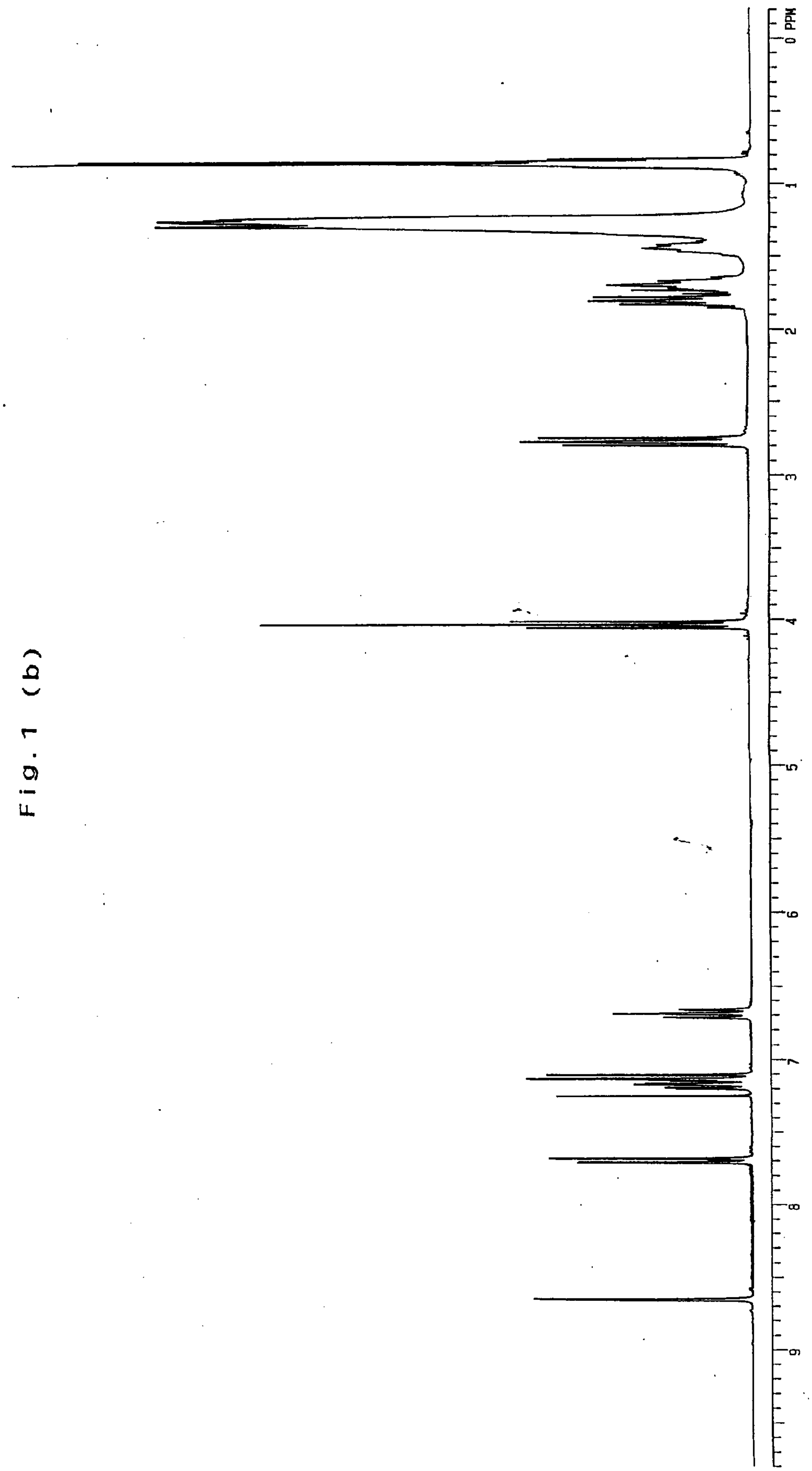

Fig. 1 (c)

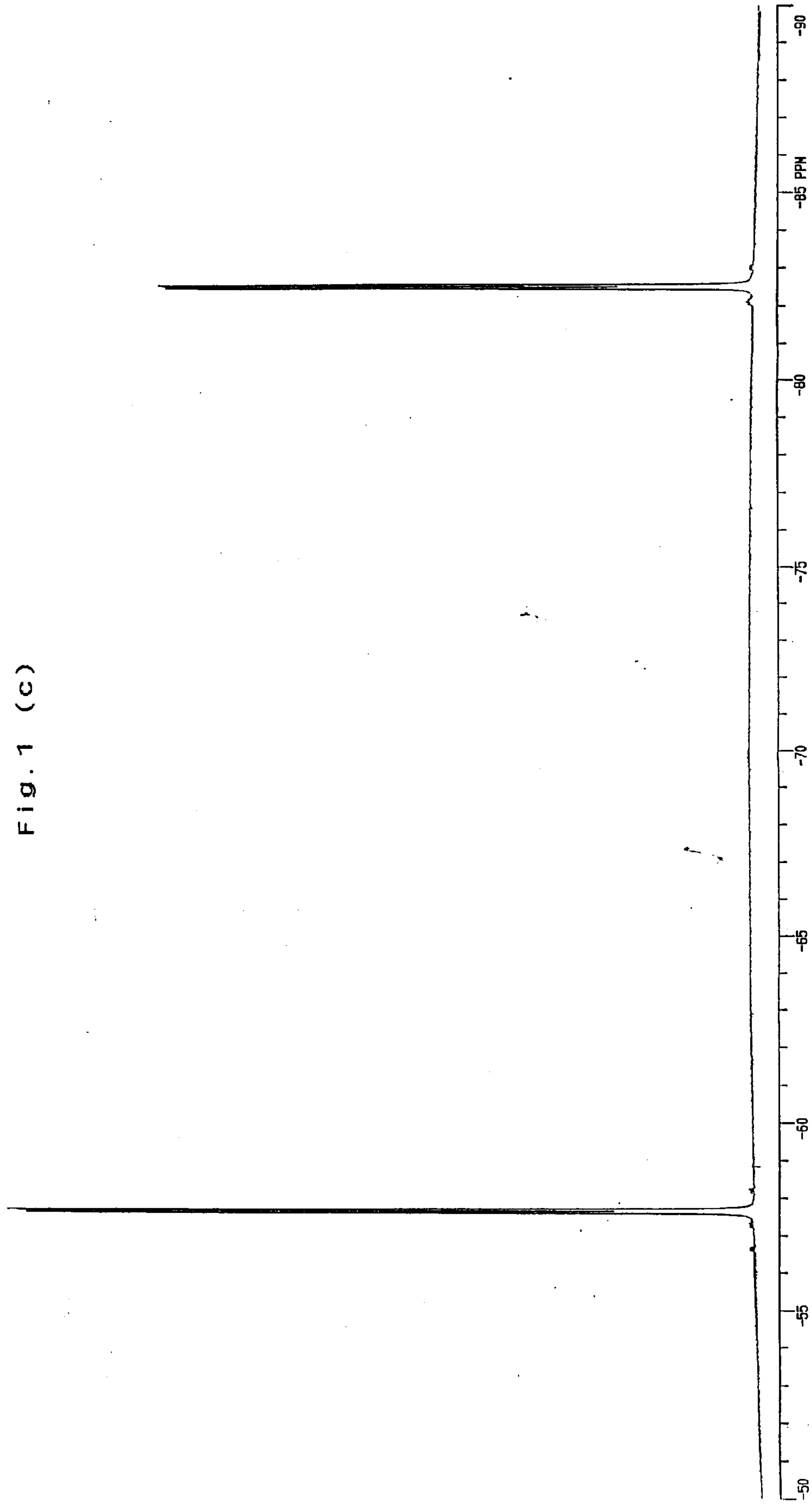

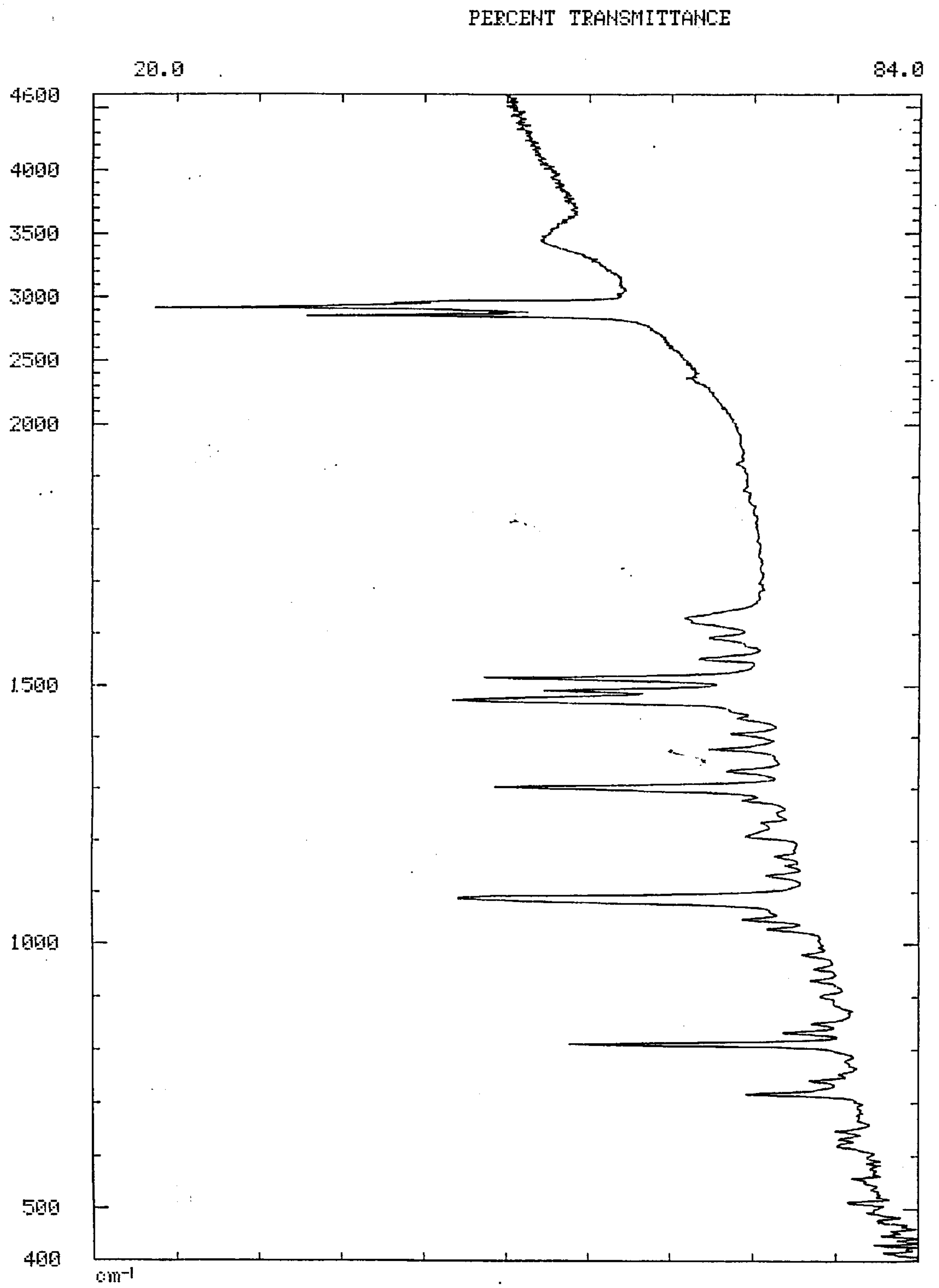
PERCENT TRANSMITTANCE
20.0
84.0
4600
4000
3500
3000
2500
2000
1500
1000
500
400
cm-1

Fig.2 (a)

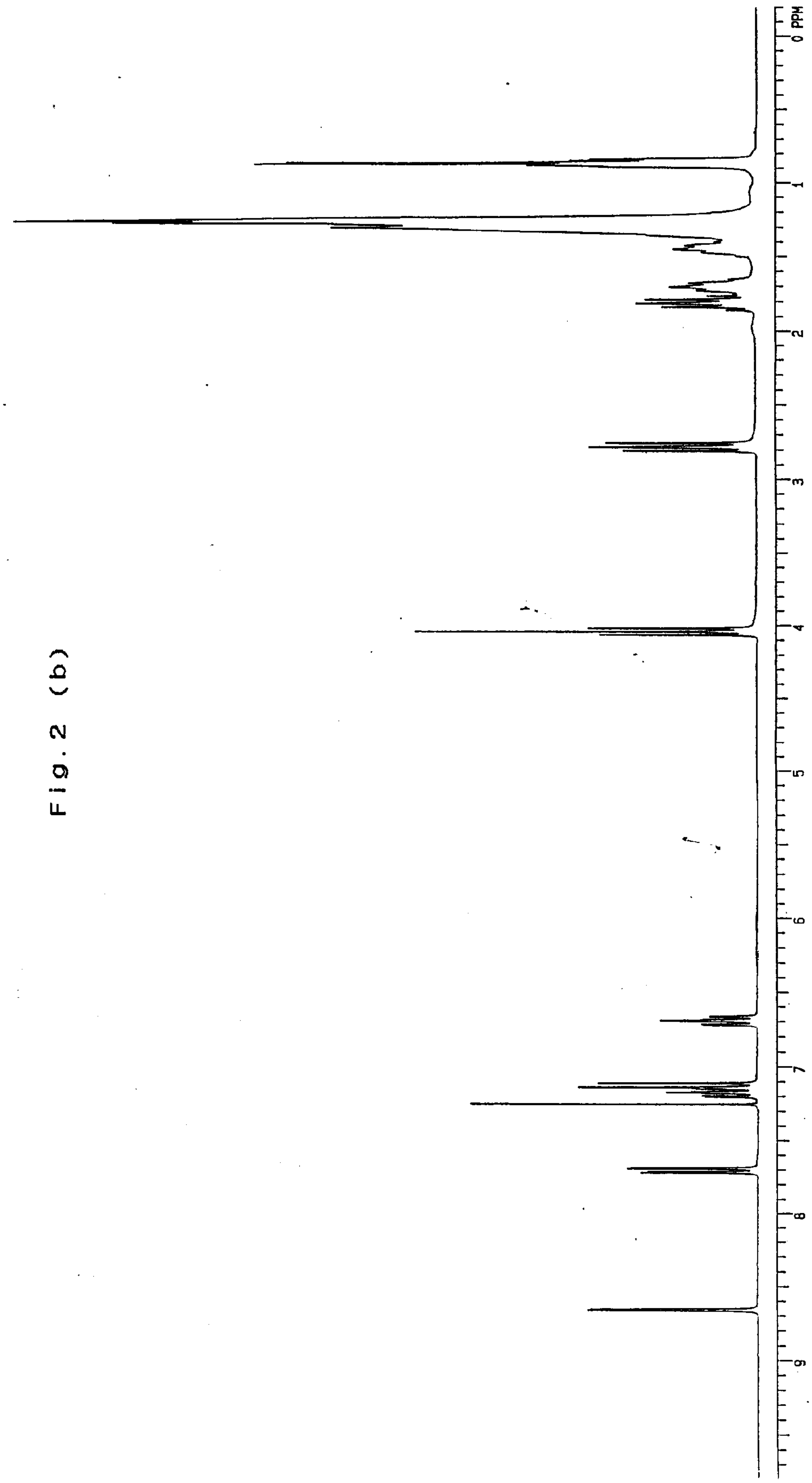


Fig.2 (b)

Fig.2 (c)

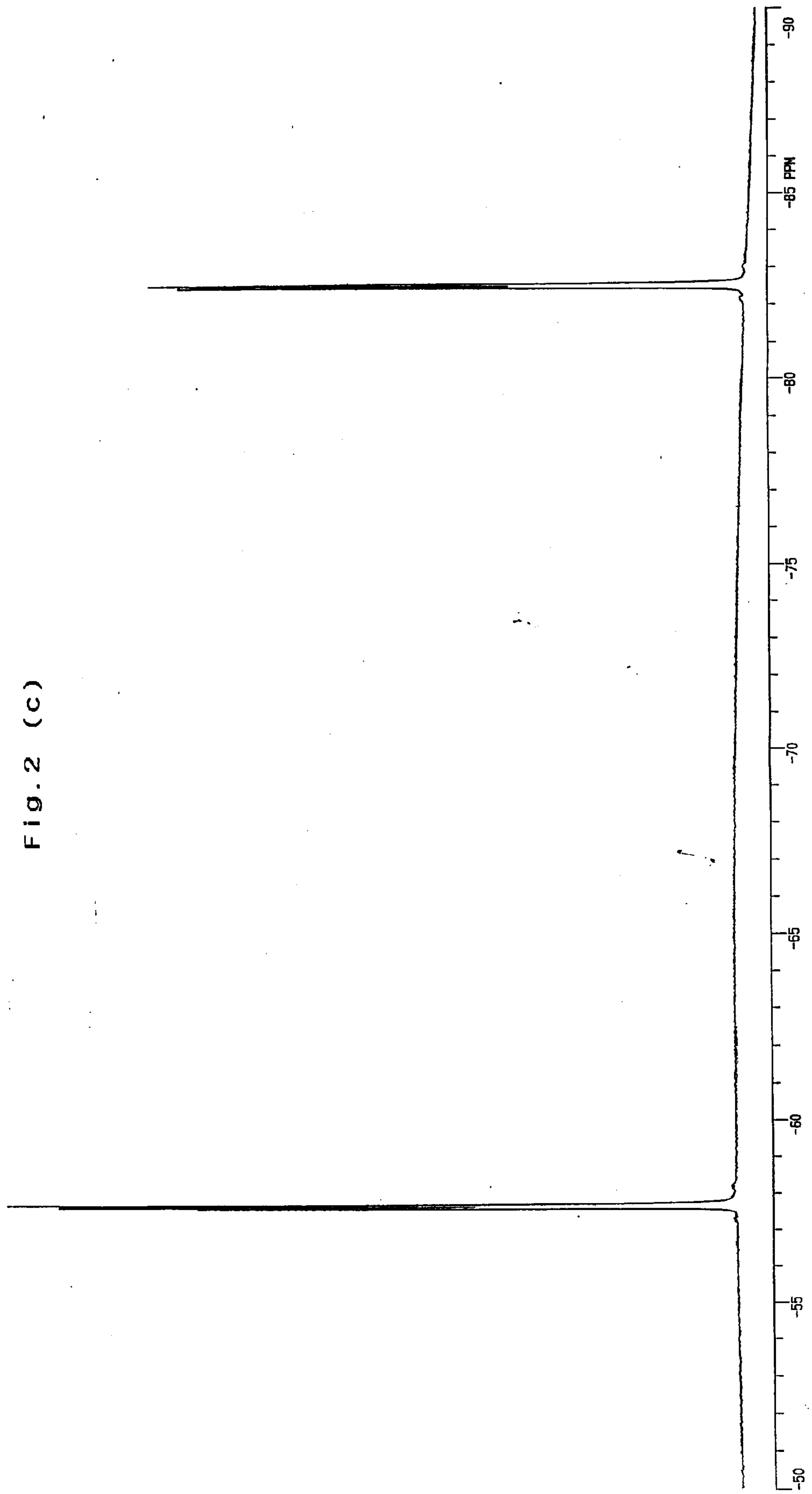

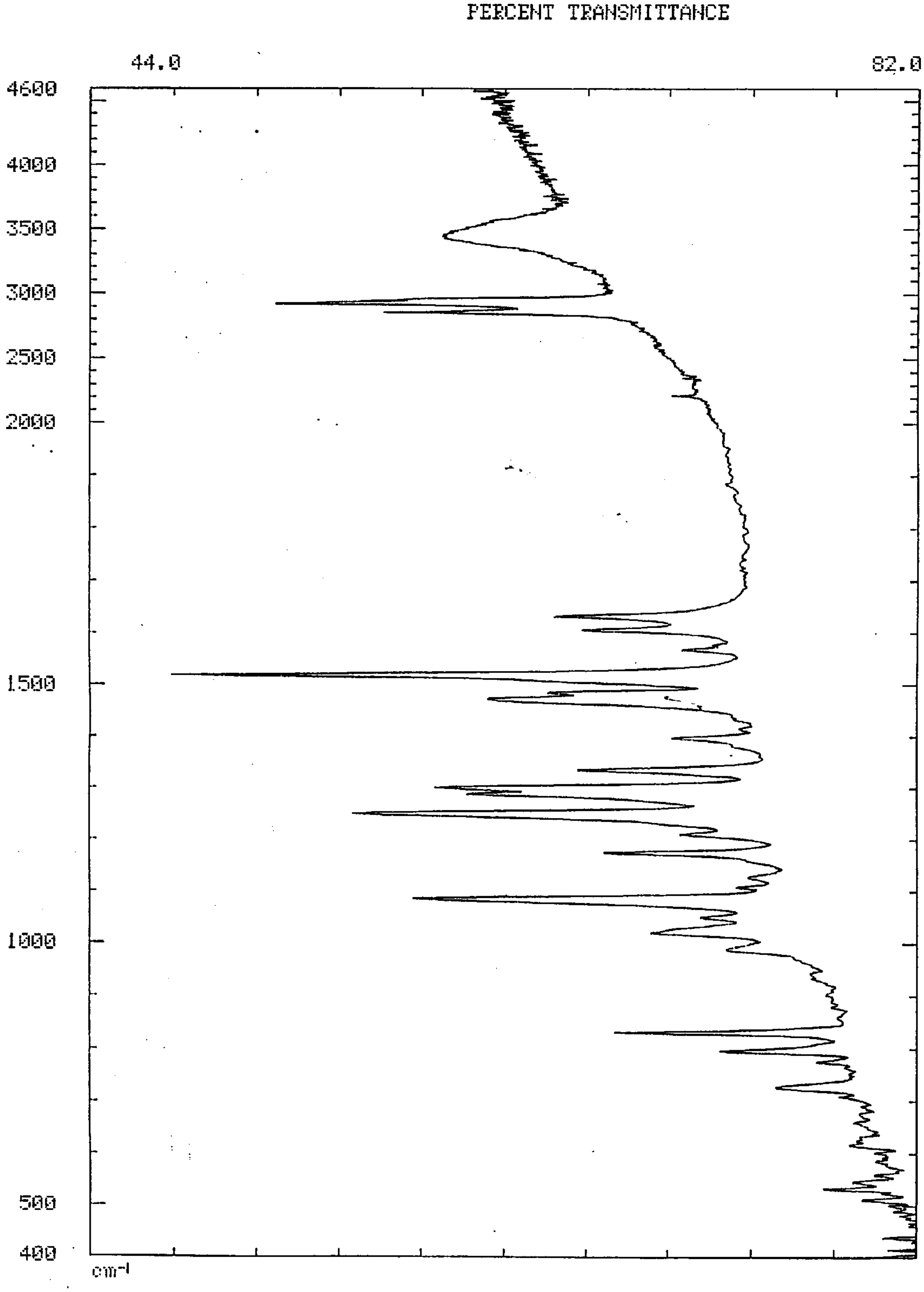
PERCENT TRANSMITTANCE
44.0
82.0
4600
4000
3500
3000
2500
2000
1500
1000
500
400
cm-1

Fig. 3 (a)

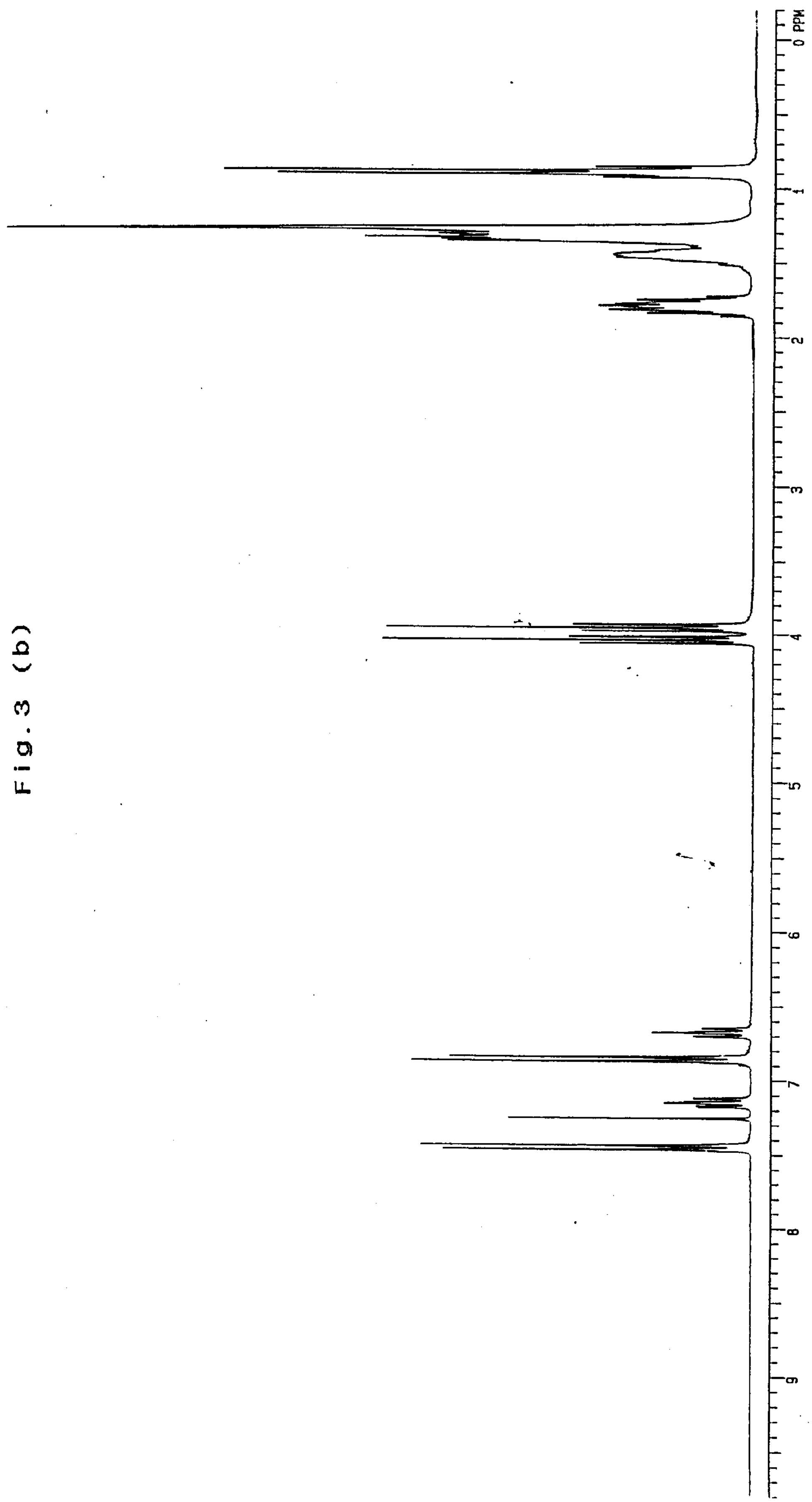


Fig.3 (b)

Fig. 3 (c)

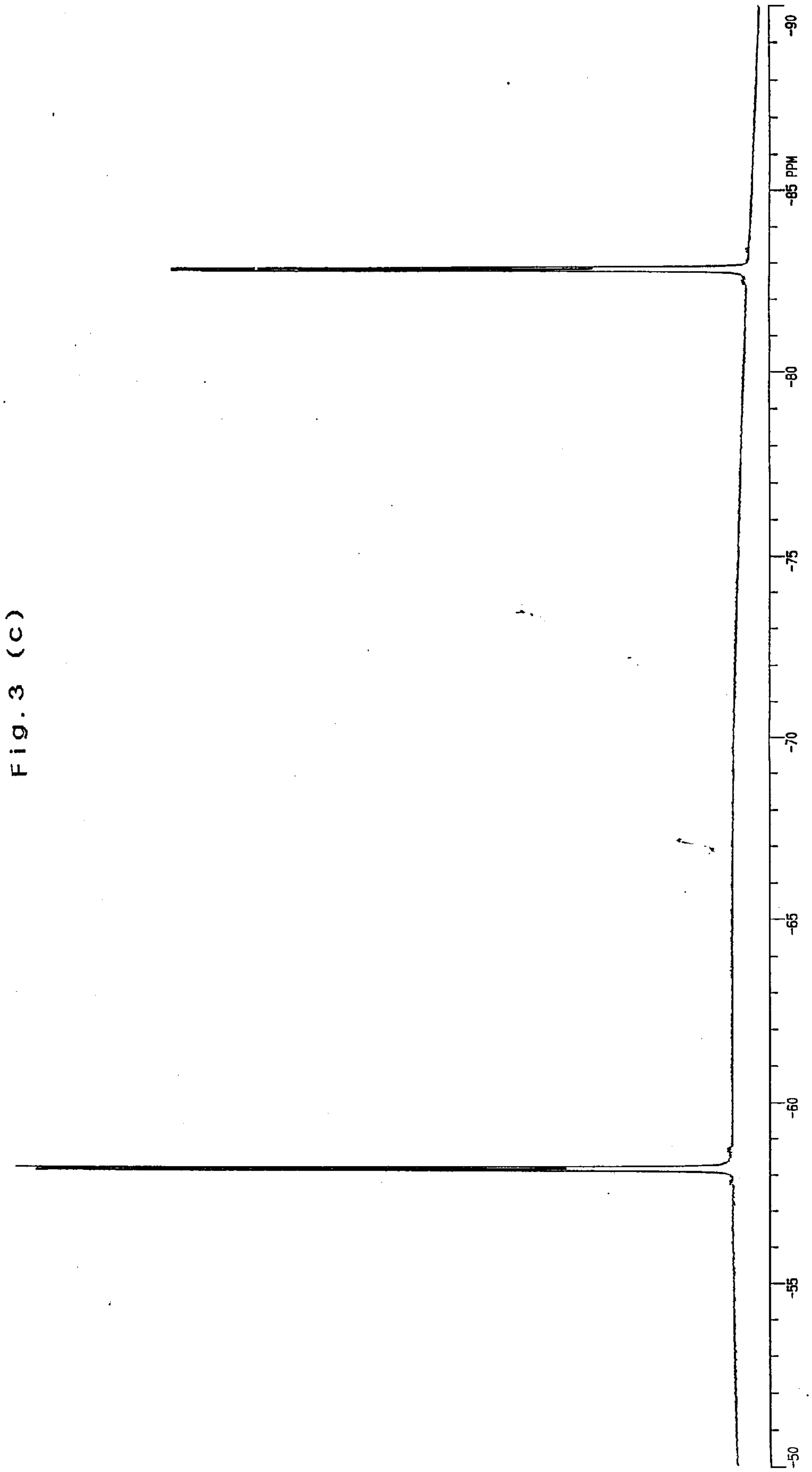

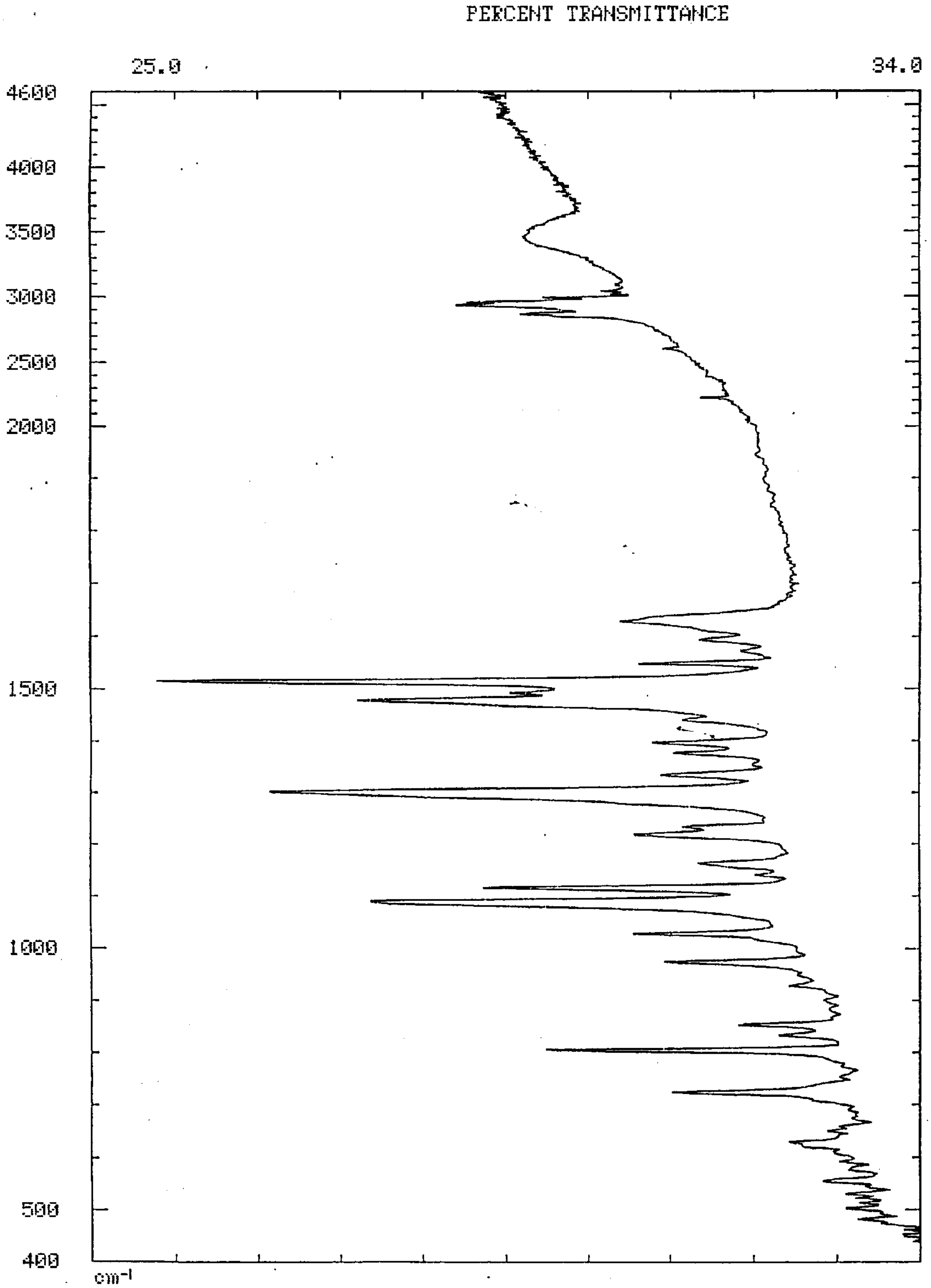
PERCENT TRANSMITTANCE
25.0
34.0
4600
4000
3500
3000
2500
2000
1500
1000
500
400
cm-1

Fig. 4 (a)

Fig.4 (b)

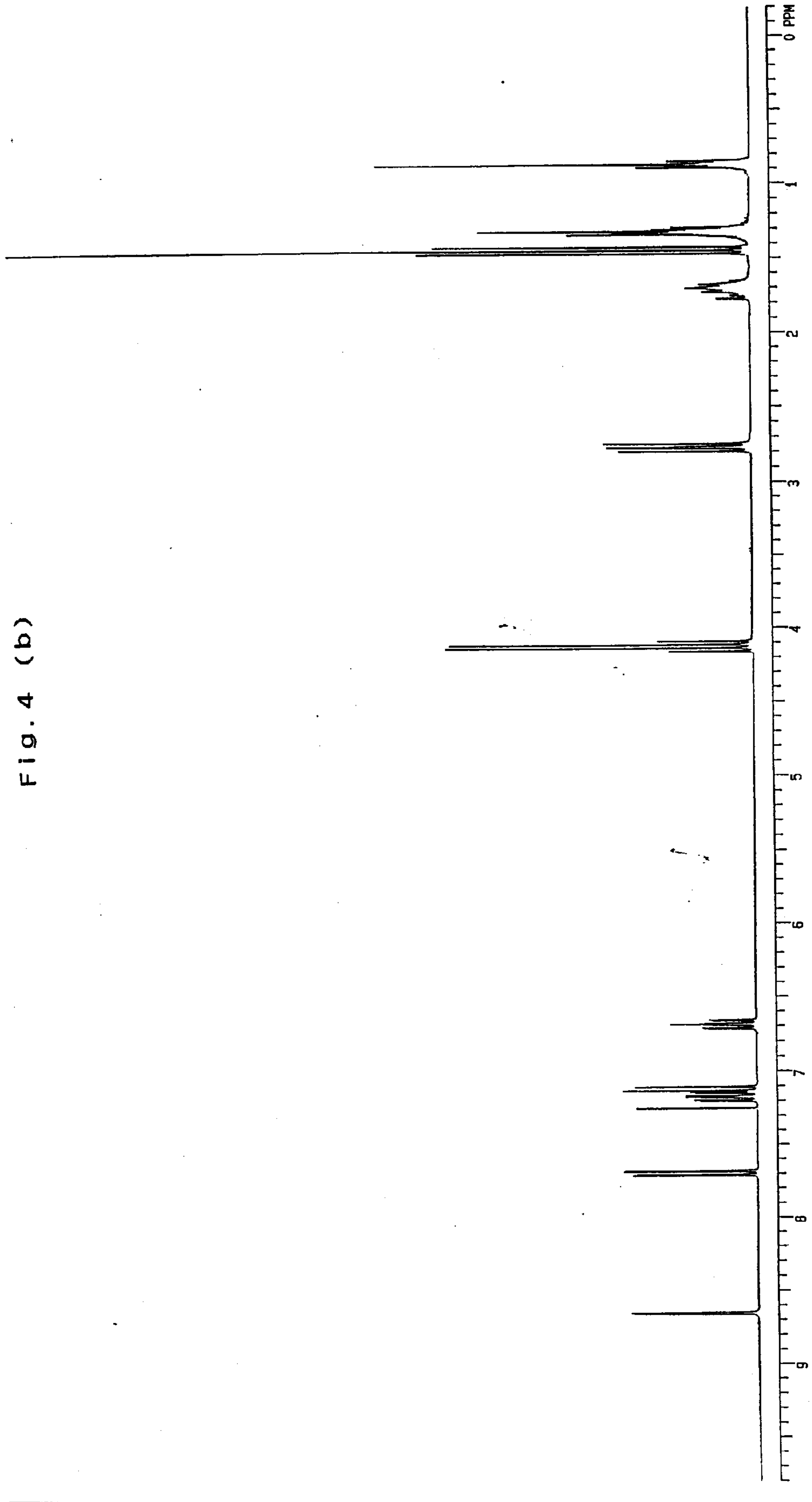

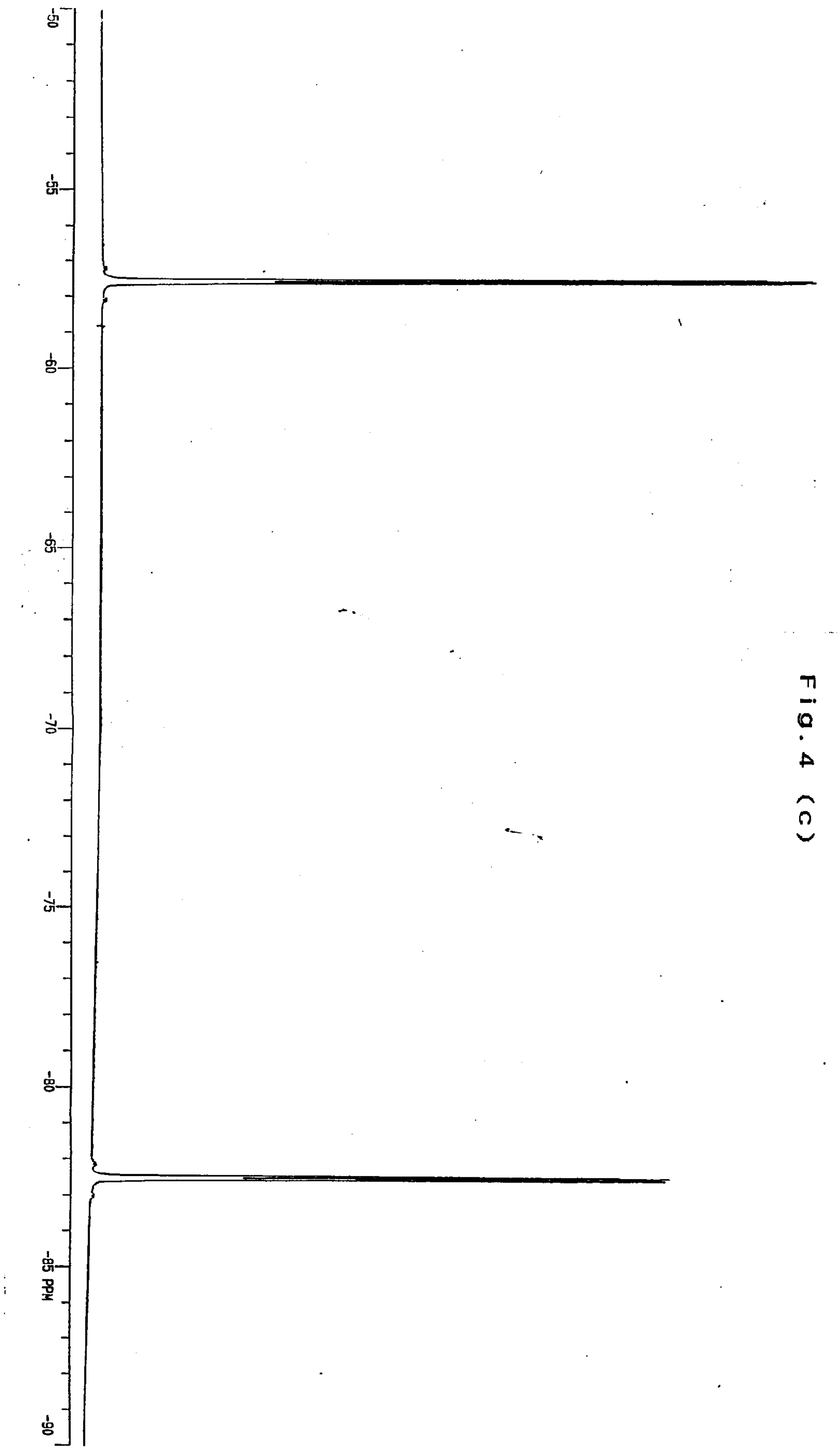


Fig.4 (c)